# EUROPÄISCHE PATENTSCHRIFT

(11) **EP 0 452 680 B1**
(45) Veröffentlichungstag und Bekanntmachung des Hinweises auf die Patenterteilung: **06.11.1996**
(21) Anmeldenummer: 91104126.7
(22) Anmeldetag: 18.03.1991
(51) Int. Cl.: C07D 473/00, C07D 473/04, C07D 473/16, C07D 473/18, C07D 473/24, C07D 473/32, A61K 31/52

(54) **Substituierte Purine, Verfahren zu ihrer Herstellung sowie ihre Verwendung als antivirale Mittel**
Substituted purines, process for their preparation and their use as antiviral agents
Purines substituées, procédé pour leur préparation et leur utilisation comme agents antiviraux

(30) Priorität: 20.03.1990 DE 4008858
(43) Veröffentlichungstag der Anmeldung: 23.10.1991
(73) Patentinhaber: HOECHST AKTIENGESELLSCHAFT, 65926 Frankfurt am Main (DE)
(72) Erfinder: Jähne, Gerhard, Dr., W-6230 Frankfurt am Main (DE); Rösner, Manfred, Dr., W-6239 Eppstein/Taunus (DE); Winkler, Irvin, Dr., W-6237 Liederbach (DE); Helsberg, Matthias, Dr., W-6233 Kelkheim/Taunus (DE); Scholl, Thomas, Dr., W-5300 Bonn (DE)

(56) Entgegenhaltungen:
- EP-A- 0 165 164
- EP-A- 0 217 207
- DE-A- 2 539 963
- CHEMICAL ABSTRACTS, Band 102, Nr. 17, 29 April 1985, Seite 612, Zusammenfassung Nr. 149282a.
- CHEMICAL ABSTRACTS, Band 98, Nr. 17, 25 April 1983, Seite 617, Zusammenfassung Nr. 143787w, Columbus, Ohio, US; H. GRIENGL et al.: "Cyclic aldehyde derivatives as alkylating reagents. I: Acyclo and azaacyclo analogs of nucleosides".
- CANADIAN JOURNAL OF CHEMISTRY, Band 62, Nr. 12, Dezember 1984, Seiten 2702-2706, Ottawa, CA; K.K. OGILVIE et al.: "Synthesis of antiviral compounds. Preparation and rearrangement of 6-methoxyglyceropurines".
- CANADIAN JOURNAL OF CHEMISTRY, Band 62, Nr. 2, Februar 1984, Seiten 241-252, Ottawa, CA; K.K. OGILVIE et al.: "Synthesis of a purine acyclonucleoside series having pronounced antiviral activity. The glyceropurines."
- JOURNAL OF HETEROCYCLIC CHEMISTRY, Band 23, Mätz/April 1986, Seiten 289-319; C.K. CHU et al.: "Chemistry and antiviral activities of acyclonucleosides".
- NUCLEOSIDES & NUCLEOTIDES, Band 8, Nr. 3, Seiten 431-448; J.L. SESSLER et al.: "The synthesis of 2-amino 7-substituted purines".
- JOURNAL OF HETEROCYCLIC CHEMISTRY, Band 23, März/April 1986, Seiten 625-627; J. KJELLBERG et al.: "Regioselective alkylation of guanine via diacycloxyglyoxal-N-acetylguanine adduct to obtain 7-alkylguanine derivatives. Studies on alkylation of guanine I"

## Beschreibung

Die vorliegende Erfindung betrifft Derivate des Purins, die in 7-Stellung einen Alkoxymethylrest tragen, Verfahren zur Herstellung dieser Verbindungen sowie ihre Verwendung als antivirale Mittel.

Im besonderen betrifft die Erfindung 2-Aminopurine, die in 7-Stellung einen unsubstituierten oder acyl- und/oder alkylsubstituierten 2-Hydroxyethoxymethyl-Rest oder 1,3-Dihydroxy-2-propoxymethyl-Rest tragen.

Ferner betrifft die Erfindung die physiologisch verträglichen Salze der genannten Verbindungen.

Während die antivirale Wirksamkeit und die Herstellung von Purinnukleosiden, die in 9-Stellung einen acyclischen Rest tragen, schon lange bekannt sind (siehe z. B. DE-OS 2539963 oder K.K.Ogilvie et al., Can.J.Chem. 62, 241 (1984) oder C.K.Chu und S.J.Cutler, J. Heterocyclic Chem. 23, 289 (1986)), ist über eine gezielte Synthese von in 7-Stellung substituierten acyclischen Purinen oder deren antivirale Wirksamkeit bisher nichts bekannt.

Lediglich J. Kjellberg et al., J.Heterocyclic Chem. 23, 625 (1986) und J. L. Sessler et al., Nucleosides + Nucleotides 8, 431 (1989) beschreiben eine mehr oder weniger selektive Methode zur Herstellung von in 7-Stellung substituierten carboacyclischen Guaninen bzw. 2-Amino-purinen. Die so dargestellten Verbindungen wurden jedoch nicht auf ihre antivirale Wirksamkeit untersucht bzw. waren bei in vitro-Untersuchungen unwirksam.

In einzelnen Fällen wurden die in 7- Stellung substituierten acyclischen Purinderivaten von den gewünschten in 9-Stellung substituierten acyclischen Purinderivaten abgetrennt und auf ihre antivirale Wirksamkeit in vitro untersucht (K. K. Ogilvie et al., Can. J. Chem. 62, 2702 (1984), K. K. Ogilvie et al., Can. J. Chem. 62, 241 (1984)) und für unwirksam befunden.

Es wurde nun überraschenderweise gefunden, daß bestimmte 7-substituierte Purine und deren physiologisch verträgliche Salze, antivirale Eigenschaften gegen verschiedene DNA-, RNA- und Retroviren aufweisen.

Erfindungsgegenstand sind demzufolge Verbindungen der Formel I 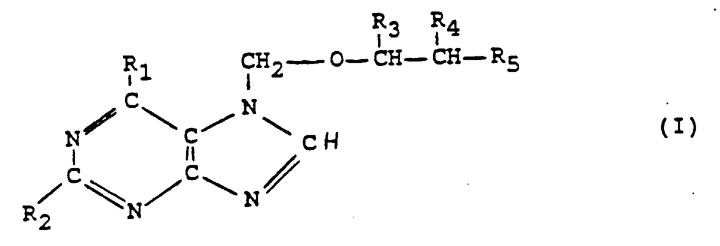 in denen
- R₁: Wasserstoff bedeutet,
- R₂: Amino bedeutet,
- R₃: C1-C3-Alkyl gegebenenfalls substituiert mit Hydroxy oder mit C1-C4-Acyloxy oder mit C1-C4-Alkoxy bedeutet,
- R₄: Hydroxy oder C1-C4-Acyloxy oder C1-C4-Alkoxy bedeutet,
und
- R₅: Wasserstoff bedeutet, insbesondere die Verbindung der Formel I, in der
- R₁: Wasserstoff bedeutet,
- R₂: Amino bedeutet,
- R₃: Hydroxymethyl bedeutet,
- R₄: Hydroxy bedeutet und
- R₅: Wasserstoff bedeutet.

Die als Substituenten der obengenannten Formel I genannten Alkylgruppen können verzweigt, unverzweigt oder cyclisch sein. Beispielhafte Alkylgruppen sind die Methyl-, Ethyl-, Propyl-, Isopropylgruppe Beispielhafte Alkoxygruppen sind die Methoxy, Ethoxy, Propoxy, Isopropoxy, Butoxygruppe.

Die Verbindungen dieser Erfindung sind allesamt substituierte acyclische Purinnukleoside, welche den acyclischen Substituenten in der 7-Stellung des Purinringsystems tragen.

Besonders für therapeutische Zwecke geeignete Salze der erfindungsgemäßen Verbindungen sind Salze von physiologisch verträglichen organischen und anorganischen Säuren wie Essigsäure, Milchsäure, Äpfelsäure, p-Toluolsulfonsäure, Methansulfonsäure, Isäthionsäure, Salzsäure oder Schwefelsäure.

Von den erfindungsgemäßen Verbindungen der Formel I werden 2-Amino-7-(1,3-dihydroxy-2-propoxymethyl)purin = Verbindung der Formel I, worin R₁ = H, R₂ = NH₂, R₃ = CH₂-OH, R₄ = OH, R₅ = H ist (Beispiel 6.12.), 2-Amino-7-(1-hydroxy-3-isopropoxy-2-propoxymethyl)purin = Verbindung der Formel I, worin R₁= Wasserstoff, R₂= Amino, R₃= Hydroxymethyl, R₄= Isopropoxy und R₅= Wasserstoff ist (Beispiel 6.10.)
und
2-Amino-7-(1,3-bis-(isopropoxy)-2-propoxymethyl)purin = Verbindung der Formel I, worin R₁ = H, R₂ = NH₂, R₃ = CH₂-O-CH(CH₃)₂, R₄ = O-CH(CH₃)₂, R₅ = H (Beispiel 6.7.) besonders bevorzugt, insbesondere wegen ihrer besonders hohen antiviralen Aktivität gegen Herpesviren.

Weiterhin zeigen andere Verbindungen der Formel I mit R₁ = Wasserstoff, R₂ = Amino und einer acyclischen Seitenkette, deren Hydroxyfunktion oder Hydroxyfunktionen mit C1-C4-Alkylresten verethert oder mit C1-C4-Acylresten verestert sind, besonders hohe antivirale Aktivität.

Zum Erfindungsgegenstand gehört weiterhin die Verwendung der genannten Verbindungen als antivirale Mittel. Besonders wirksam sind die erfindungsgemäßen Verbindungen gegen Herpes Simplex Viren Typ 1 und Typ 2, Cytomegalie Viren, Varicella Zoster Viren, Epstein Barr Viren und Human Herpes Viren 6 (HHV6)

Weiterhin betrifft die vorliegende Erfindung Verfahren zur Herstellung von substituierten Purinen der Formel I oder eines physiologisch verträglichen Salzes derselben, dadurch gekennzeichnet, daß man
1) wenn in der Verbindung der Formel I R₄ Hydroxy ist, eine Schutzgruppe (blockierende Gruppe) A₁ in einer Verbindung der Formel II durch eine Hydroxygruppe ersetzt, 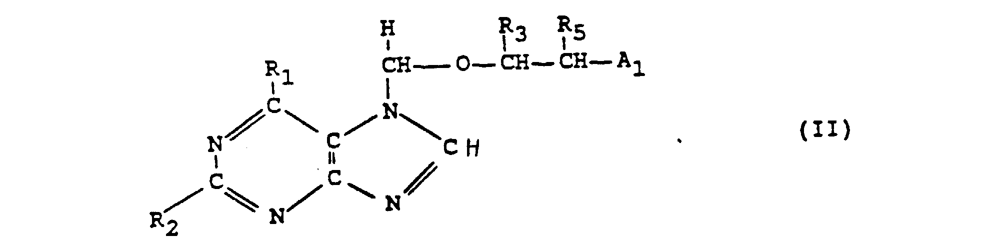
2) wenn in der Verbindung der Formel I R₃ Hydroxyalkyl ist, eine Schutzgruppe A₂ in einer Verbindung der Formel III durch eine Hydroxygruppe ersetzt, 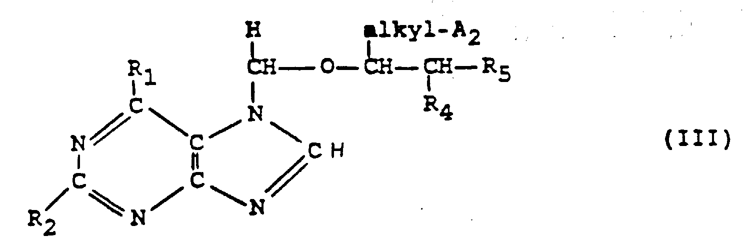
4) wenn in der Verbindung der Formel I R₃ Hydroxyalkyl ist und/oder R₄ Hydroxy ist, eine Schutzgruppe A₄ und/oder A₅ in einer Verbindung der Formel V durch eine Hydroxygruppe ersetzt, 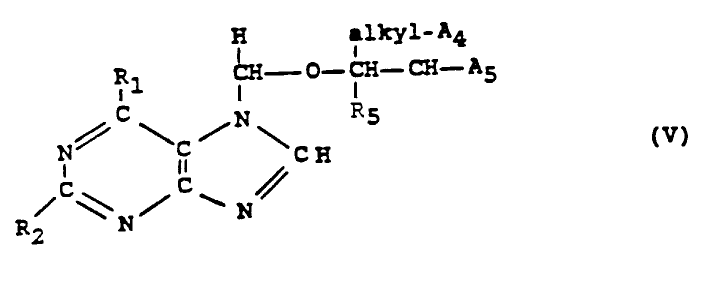
5) eine Verbindung der Formel VI, worin Y und Z Vorläufer der Gruppen R₁ bzw. R₂ sind, in eine Verbindung der Formel I umwandelt, worin R₁ und R₂ die oben beschriebenen Bedeutungen haben, 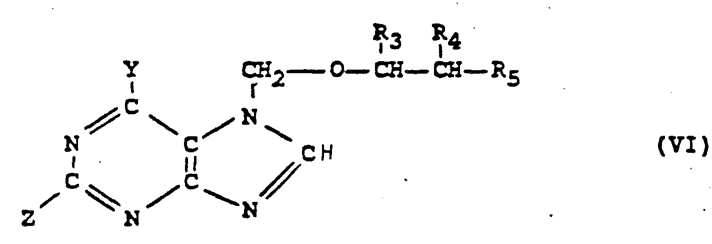
6) eine Verbindung der Formel VII 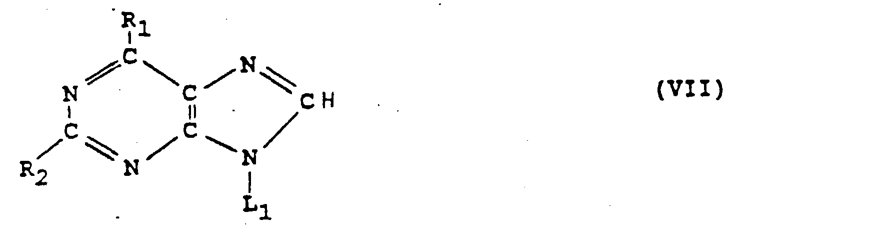 mit einer Verbindung der Formel VIII 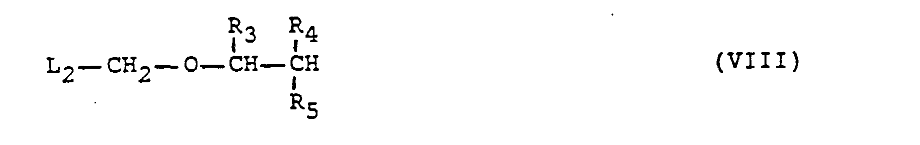 umsetzt, worin L₂ eine austretende Gruppe und L₁ Wasserstoff oder eine austretende Gruppe ist,
7) eine Blockierungsgruppe von einer Verbindung der Formel I, worin einer oder beide Reste R₁ und R₂ blockiert sind, entfernt, und insoweit das Produkt der Reaktion eine Base der Formel I ist, es gegebenenfalls in ein Säureadditionsprodukt dieser Base der Formel I umwandelt, oder insoweit das Produkt der Reaktion ein Salz einer Base der Formel I darstellt, es gegebenenfalls in seine Base oder in ein anderes Salz dieser Base umwandelt.

In den Fällen der Verfahren 1), 2) und 4) sind, soweit vorhanden, Hydroxyfunktionen der acyclischen Seitenkette in 7-Stellung des Purinsystems endständig durch eine blockierende Gruppe D und gegebenenfalls eine weitere blockierende Gruppe E abgewandelt, wobei D gleich oder verschieden von E sein kann.

Diese blockierende Gruppen können Ester - beispielsweise Acyloxygruppen - und/oder Benzyloxygruppen - und/oder C1-C6-Alkyloxygruppen - beispielsweise Isopropoxygruppen - sein.

Im ersten Falle kann die Acyloxygruppe aliphatisch - beispielsweise Acetoxy oder Pivaloyloxy - oder aromatisch - beispielsweise Benzoyloxy - sein.
Beide Arten von Acylgruppen können beispielsweise durch milde basische Hydrolyse entfernt werden; im allgemeinen ist ein Erwärmen mit wäßrigem oder alkoholischem Methylamin ausreichend, um eine Entfernung der blockierenden Gruppe zu erreichen.

Im zweiten Falle kann die Benzyloxyblockierungsgruppe durch Hydrogenolyse entweder katalytisch oder mittels Wasserstoff und Raney-Nickel oder Palladium/ Kohle oder mittels Ammoniumformiat und Palladium/Kohle oder mittels einer Transferhydrogenolyse mit Palladiumhydroxid und Cyclohexen oder Cyclohexadien oder chemisch durch Reaktion mit Borhalogeniden - beispielsweise Bortrichlorid - bei tiefen Temperaturen - beispielsweise bei -70 Grad Celsius - oder mittels Natrium in flüssigem Ammoniak, wobei der flüssige Ammoniak als Lösungsmittel dient, entfernt werden.

Im Falle der katalytischen Hydrogenolyse ist das bevorzugte Lösungsmittel ein Alkanol; wobei jedoch ein Reihe von inerten Lösungsmitteln auch Verwendung finden können, sofern das Substrat wenigstens teilweise darin löslich ist. Beispiele dafür sind Benzol, Toluol, Tetrahydrofuran oder Dioxan.
Für die chemische Reaktion mittels Bortrichlorid, wobei man eine Lösung von Bortrichlorid in n-Hexan oder in Dichlormethan oder aber gasförmiges Bortrichlorid benutzt, ist Dichlormethan das bevorzugte Lösungsmittel.

Im dritten Falle ist eine Entblockierung der Cl-C6-Alkyloxygruppen dann zu erreichen, wenn man Bortrihalogenide - beispielsweise Bortrichlorid - bei weniger tiefen Temperaturen - beispielsweise bei -60°C bis 0°C, bevorzugt bei -40°C bis -20°C - mit dem Substrat zur Reaktion bringt. Das bevorzugte Lösungsmittel dafür ist Dichlormethan und das Bortrichlorid kann gasförmig, als Lösung in n-Hexan oder als Lösung in Dichlormethan eingesetzt werden.

Die Umwandlung einer Verbindung der Formel VI in eine Verbindung der Formel I nach dem Verfahren 5) kann in sehr unterschiedlicher Weise erreicht werden.
Beispielsweise kann einer der beiden Reste R₁ oder R₂ in eine Aminogruppe durch Ammonolyse, in eine Aminogruppe durch Entblockierung einer C1-C8-Acylamino-, C1-C8-Thioacylamino-, Benzylamino- oder C1-C6-Alkylaminogruppe, in Wasserstoff durch Hydrogenolyse oder Desulfurierung oder Bildung des Azids überführt werden.

Sämtliche dieser Verfahren sind bekannt und beispielsweise zu finden in: Heterocyclic Compounds - Fused Pyrimidines Part II, Purines, Herausgeber: D.J.Brown, verlegt bei Wiley-Interscience, 1971.

In dem Verfahren 6) ist die austretende Gruppe L₂ einer Verbindung der Formel VIII entweder ein reaktionsfähiger Rest einer anorganischen Säure und kann somit
a) Halogen , bevorzugt Chlor, sein, oder
b) eine C1-C6-Alkylthio- oder C1-C6-Alkylsulfinyl- oder C1-C6-Alkylsulfonyl-Gruppierung , bevorzugt die Methylthio- oder die Methylsulfinyl- oder die Methylsulfonylgruppierung sein, oder sie ist ein reaktionsfähiger Rest einer organischen Säure und kann somit
c) C1-C8-Acyloxy oder Benzoyloxy, bevorzugt Acetoxy, sein.

In dem Verfahren 6a) ist die Abgangsgruppe L₁ in einer Verbindung der Formel VII Wasserstoff oder Trialkylsilyl, bevorzugt Trimethylsilyl.

In dem Verfahren 6b) ist die Abgangsgruppe L₁ in einer Verbindung der Formel VII eine C1-C8-Acyloxygruppe, bevorzugt die Acetoxygruppe, oder eine Trialkylsilylgruppe, bevorzugt die Trimethylsilylgruppe.

In dem Verfahren 6c) ist die Abgangsgruppe L₁ in einer Verbindung der Formel VII eine C1-C8-Acyloxygruppe oder aber bevorzugt eine Trialkylsilylgruppe, im besonderen die Trimethylsilylgruppe.

Das bevorzugte Verfahren nach 6a) beinhaltet die Kondensation eines Purins mit der gewünschten Substitution in 2-Stellung mit einem Acyl- und/oder Alkyl-blockierten 2-(Halogenmethoxy)-1,3-propandiol, beispielsweise 2-(Chlormethoxy)-1,3-bis(acetoxy)-propan oder in einem stark polaren Lösungsmittel wie Dimethylformamid Dimethylacetamid, N-Methyl-pyrrolidon-(2), Tetramethylharnstoff oder Dimethylsulfoxid, in Gegenwart einer Base, wie Triethylamin, N-Ethylmorpholin oder eines Alkalicarbonats, wie beispielsweise Kaliumcarbonat, bei Raumtemperatur während 1 - 72 Stunden, wenn L₁ in einer Verbindung der Formel VII Wasserstoff ist, oder
in aprotischen Lösungsmitteln wie Benzol, Toluol, Xylol, 1,2- Dichlorethan, Chlorbenzol, 1,2-Dimethoxyethan, Dioxan oder Acetonitril,
in Gegenwart einer Base wie Triethylamin oder N-Ethylmorpholin, bei einer Reaktionstemperatur von 0 bis 150°C, vorzugsweise bei Raumtemperatur, während 1 - 72 Stunden, wenn L₁ in einer Formel der Verbindung VII Trimethylsilyl ist.

Es ist bekannt, daß Alkylthioalkylether, im besonderen Methylthiomethylether wie beispielsweise Verbindungen der Formel VIII mit L₂ = Methylthio, mit Sauerstoffnukleophilen und Lewis-Säuren wie Quecksilber-(II)-chlorid (E.J.Corey, M.G.Bock, Tetrahedron Letters 1975, 3269 oder K.Yamada, K.Kato, H.Nagase, Y.Hirata, Tetrahedron Letters 1976, 65) oder Alkylsulfinylalkylether, im besonderen Methylsulfinylmethylether wie beispielsweise Verbindungen der Formel VIII mit L₂ = Methylsulfinyl, mit Kohlenstoffnukleophilen und Lewis-Säuren wie Zinkjodid (J.A.Schwindeman, P.D.Magnus, Tetrahedron Letters 1981, 4925) zur Reaktion gebracht werden können.

Das bevorzugte Verfahren nach 6b) beinhaltet die Kondensation eines Purins mit der gewünschten Substitution in 2-Stellung mit einem C1-C4-Acyl- und/oder C1-C4-Alkylblockierten 2-(Alkylthioalkoxy)-1,3-propandiol, beispielsweise 2-(Methylthiomethoxy)-1,3-bis(acetoxy)-propan oder 2-(Methylthiomethoxy)-1,3-bis(isopropoxy)-propan,
wobei jeweils anstelle der Alkylthioalkoxyqruppierung auch die Alkylsulfinylalkoxy- oder
Alkylsulfonylalkoxygruppierung vorteilhaft eingesetzt werden kann,
in einem stark polaren Lösungsmittel oder Lösungsmittelgemisch wie Dimethylformamid, Dimethylacetamid, N-Methylpyrrolidon-(2), Tetramethylharnstoff und/oder Dimethylsulfoxid, in Gegenwart einer Protonsäure oder Lewis-Säure, wie Eisentrichlorid, Bortrifluorid, Galliumtrichlorid, Aluminiumtrichlorid, Titantetrachlorid, bevorzugt jedoch Zinntetrachlorid, oder Jod oder
Trifluormethansulfonsäuretrialkylsilylester, bevorzugt Trifluormethansulfonsäuretrimethylsilylester, bei einer Temperatur von -40°C bis +100°C, vorzugsweise zwischen -20°C und +80°C während mehrerer Stunden, wenn L₁ in einer Verbindung der Formel VII C1-C8-Acyl, vorzugsweise Acetyl ist, oder
in einem weniger polaren Lösungsmittel oder Lösungsmittelgemisch wie Dichlormethan oder 1,2-Dichlorethan
in Gegenwart einer Lewis-Säure wie Eisen-III-chlorid, Bortrifluorid, Galliumtrichlorid, Aluminiumtrichlorid, Titantetrachlorid oder Zinntetrachlorid oder eines Trifluormethansulfonsäuretrialkylsilylester, bevorzugt Trifluormethansulfonsäuretrimethylsilylester,
bei einer Temperatur von -40°C bis +100°C, vorzugsweise zwischen -30°C und +20°C während 0.5 bis 8 Stunden, vorzugsweise während 1 bis 4 Stunden, wenn
L₁ in einer Verbindung der Formel VII Trialkylsilyl, vorzugsweise Trimethylsilyl, ist, oder
in einem polaren aprotischen Lösungsmittel wie Acetonitril in Gegenwart einer Lewis-Säure wie Eisen-III-chlorid, Bortrifluorid, Galliumtrichlorid, Aluminiumtrichlorid, Titantetrachlorid, bevorzugt jedoch Zinntetrachlorid bei einer Temperatur von -40°C bis +100°C, vorzugsweise zwischen -30°C und +20°C, während 0.5 bis 8 Stunden, bevorzugt während 1 bis 4 Stunden, wenn
L₁ in einer Verbindung der Formel VII Trialkylsilyl, vorzugsweise Trimethylsilyl, ist.

Das bevorzugte Verfahren nach 6c) beinhaltet die Kondensation eines Purins mit der gewünschten Substitution in 2-Stellung, mit einem C1-C4-Acyl- und/oder C1-C4-Alkyl-blockierten 2-(C1-C8-Acyloxymethoxy)-1,3-propandiol, beispielsweise 2-Acetoxymethoxy-1,3-bis (acetoxy)-propan oder 2-Acetoxymethoxy-1,3-bis(isopropoxy)-propan, in einem aprotischen Lösungsmittel wie Benzol, Toluol, Xylol, Acetonitril, Dichlormethan oder 1,2-Dichlorethan oder Gemischen davon,
in Gegenwart einer Säure, bevorzugt einer Lewis-Säure wie Aluminiumtrichlorid, Bortrifluorid, Eisentrichlorid, Galliumtrichlorid, Zinntetrachlorid oder Titantetrachlorid oder in Gegenwart von Jod
oder vorzugsweise
Trifluormethansulfonsäuretrialkylsilylester, besonders Trifluormethansulfonsäuretrimethylsilylester, wobei die Mengen dieser Reagenzien 0.1 bis 10, vorzugsweise 0.8 bis 7 Äquivalente, bezogen auf die Menge der jeweils eingesetzten Acetoxymethoxyverbindung, betragen,
bei Temperaturen zwischen -70°C und +80°C, vorzugsweise zwischen -40°C und +30°C, während 2 bis 24 Stunden, vorzugsweise während 2 bis 6 Stunden, wenn
L₁ in einer Verbindung der Formel VII Trialkylsilyl, besonders Trimethylsilyl ist.

Dieses Verfahren liefert in hoher Regioselektivität, in der Regel >>9:1, bevorzugt das 7-Isomer des jeweiligen acyclischen Purinderivates.

Entstehen nach den Verfahren 6a) - 6c) Produktgemische, so werden diese, eventuell nach Umwandlung in ein anderes Purinderivat, chromatographisch oder durch fraktionierte Kristallisation in die reinen Komponenten aufgetrennt.

Verbindungen der Formel VIII mit L₂ = Halogen können hergestellt werden, indem man ein zweckmäßig modifiziertes und geschütztes Alkanol, beispielsweise 1,3-Bis(acetoxy)-propan-2-ol oder 1,3-Bis(isopropoxy)-propan-2-ol mit Paraformaldehyd und einem gasförmigen Halogenwasserstoff, beispielsweise Chlorwasserstoff, in einem inerten Lösungsmittel, beispielsweise Dichlormethan, bei Raumtemperatur oder darunter umsetzt.

Die Darstellung von Halogenmethylethern ist eine allgemein anwendbare Reaktion; eine ausführliche Beschreibung findet man beispielsweise in: Houben - Weyl, Methoden der organischen Chemie Georg Thieme Verlag, Stuttgart, 1965, Band VI/3, S.125ff.

Verbindungen der Formel VIII mit L₂ = Methylthio können hergestellt werden, indem man ein zweckmäßig modifiziertes und geschütztes Alkanol, beispielsweise 1,3-Bis(acetoxy)-propan-2-ol oder 1,3-Bis(isopropoxy)-propan-2-ol mit Dimethylsulfoxid, C1-C8-Säureanhydrid und C1-C8-Carbonsäure in einem Temperaturbereich zwischen 0 °C und +40°C, vorzugsweise bei Raumtemperatur, während mehrerer Tage, in der Regel 2 bis 4 Tage, umsetzt.

Eine ausführliche Beschreibung zur Herstellung von Methylthiomethylethern primärer, sekundärer und tertiärer Alkohole findet sich bei P.M.Pojer und S.J.Angyal, Aust.J.Chem., 31, 1031 (1978).

Die entsprechenden Methylsulfinylmethylverbindungen und Methylsulfonylmethylverbindungen können durch Oxidation mittels Persäuren, beispielsweise m-Chlorperbenzoesäure oder Peressigsäure, in einfacher Weise erhalten werden.

Verbindungen der Formel VIII mit L₂ = C1-C8-Acyloxy können hergestellt werden, entweder aus einer Verbindung der Formel VIII mit L₂ = Halogen, welche wie oben beschrieben zugänglich ist, durch Umsetzung mit einem Alkalicarboxylat, vorzugsweise Natrium- oder Kaliumacetat, in Aceton oder Dimethylformamid, oder indem man ein zweckmäßig modifiziertes und geschütztes Alkanol in den Alkoxyalkylether, vorzugsweise den Methoxymethylether, überführt, welcher dann seinerseits durch Reaktion mit einem C1-C8-Carbonsäureanhydrid, vorzugsweise Essigsäureanhydrid, unter Proton- oder Lewis-SäureKatalyse, vorzugsweise Bortrifluorid-Etherat-Katalyse, in die C1-C8-Acyloxyverbindung, vorzugsweise die Acetoxyverbindung, überführt wird (für beide Verfahren siehe:
Houben - Weyl, Methoden der organischen Chemie, Georg Thieme Verlag, Stuttgart, 1965, Band VI/3, S. 286f).

Besonders einfach und effektiv jedoch werden die Verbindungen der Formel VIII mit L₂ = C1-C8-Acyloxy hergestellt, indem man ein zweckmäßig modifiziertes und geschütztes Alkanol, beispielsweise 1,3-Bis(acetoxy)-propan-2-ol oder 1,3-Bis(isopropoxy)-propan-2-ol mit einer Carbonsäure, vorzugsweise Essigsäure, und dem dazugehörenden-Anhydrid, vorzugsweise Essigsäureanhydrid, in Dimethylsulfoxid, wobei vorzugsweise pro 0.1 Mol Alkanol ca. 60 ml Säure, ca. 50 ml Anhydrid und ca. 100 ml Dimethylsulfoxid verwendet werden, unterhalb Raumtemperatur, vorzugsweise bei 0°C, zusammengibt, und mehrere Stunden, vorzugsweise 4 bis 6 Stunden, bei erhöhter Temperatur, vorzugsweise bei 40 bis 100 Grad Celsius, rührt.

In dem Verfahren 7) können die Substituenten R₁ und R₂ durch beispielsweise Trialkylsilylgruppen, vorzugsweise Trimethylsilylgruppen, blockiert sein.

Solcherart Verbindungen werden das Produkt der Kondensation eines per-trimethylsilylierten Purins und einer Verbindung der Formel VIII wie im Verfahren 6) sein.

Diese Blockierungsgruppen sind labil und können durch Solvolyse mit Wasser, mit wäßrigem oder alkoholischem Ammoniak oder mit wäßriger Hydrogencarbonatlösung oder durch Alkoholyse entfernt werden.

Ein weiteres Verfahren kombiniert die Verfahren 1) bzw. 2) bzw. 4) mit dem Verfahren 5); dabei kann eine Entblockierung durch Solvolyse gleichzeitig mit dem Ersatz einer aus dem Purinsystem austretenden Gruppe, beispielsweise Halogen, erzielt werden, wie beispielsweise durch die Reaktion mit flüssigem Ammoniak. In diesem Falle wird neben der Entblockierung der Seitenkette, insoweit diese mit einer C1-C8-Acyloxygruppe geschützt war (siehe Verfahren 1) , 2) und 4)), gleichzeitig die austretende Gruppe am Purinsystem durch die Aminogruppe ersetzt. Ferner kann dabei eine C1-C8-acylblockierte Gruppe am Purinsystem entblockiert werden.

Die erfindungsgemäßen Verbindungen der Formel I können in der acyclischen Seitenkette ein oder mehrere chirale Zentren aufweisen. Die Verbindungen liegen in der Regel als Racemate vor; eine Herstellung bzw. Isolierung der reinen Enantiomere ist möglich. Gegenstand der Erfindung sind deshalb sowohl die reinen Enantiomeren als auch Mischungen derselben, wie z.B. das zugehörige Racemat.

Des weiteren betrifft die vorliegende Erfindung Arzneimittel mit einem Gehalt an mindestens einer erfindungsgemäßen Verbindung.

Die erfindungsgemäßen Arzneimittel, können enteral (oral), parenteral (intravenös), rektal oder lokal (topisch) angewendet werden. Sie können in Form von Lösungen, Pulvern (Tabletten, Kapseln einschließlich Mikrokapseln), Salben (Cremes oder Gel) oder Suppositorien verabreicht werden. Als Hilfsstoffe für derartige Formulierungen kommen die pharmazeutisch üblichen flüssigen oder festen Füllstoffe und Streckmittel, Lösemittel, Emulgatoren, Gleitstoffe, Geschmackskorrigentien, Farbstoffe und/oder Puffersubstanzen in Frage. Als zweckmäßige Dosierung werden 0,1 - 10, vorzugsweise 0,2 - 8 mg/kg Körpergewicht verabreicht. Sie werden zweckmäßig in Dosierungseinheiten verabreicht, die mindestens die wirksame Tagesmenge der erfindungsgemäßen Verbindungen, z.B. 30 - 300, vorzugsweise 50 - 250 mg enthalten.

Die erfindungsgemäßen Verbindungen können auch in Kombination mit anderen Antivirusmitteln und Immunstimulantien, wie Interferonen, verabreicht werden.

In vitro Versuche und Ergebnisse:

Die antivirale Wirksamkeit der erfindungsgemäßen Verbindungen wurde in in vitro Versuchen geprüft. Dazu wurden die erfindungsgemäßen Verbindungen in verschiedenen Verdünnungen zu Zellkulturen von Vero-Zellen in Mikrotiterplatten gegeben. Nach 3-Stunden wurden die Kulturen mit unterschiedlichen Viren infiziert. Vero-Zellen wurden mit verschiedenen humanpathogenen Herpesviren infiziert, HeLa-Zellen wurden mit Vaccinia Virus und MDBK-Zellen mit Vesikular Stomatitis Virus infiziert. 48 - 72 Stunden nach der Infektion wurde der Therapieerfolg anhand des cytopathischen Effektes mikroskopisch und nach Neutralrotaufnahme (Farbtest nach Finter) photometrisch bestimmt (Finter, N.B., in "Interferons" (N.B.Finter et al.), North Holland Publishing Co., Amsterdam (1966)). Die minimale Konzentration, bei der etwa die Hälfte der Zellen keinen cytopathogenen Effekt zeigt, wird als minimale Hemmkonzentration (MHK) betrachtet. Die Ergebnisse sind in Tabelle 1 zusammengefaßt.

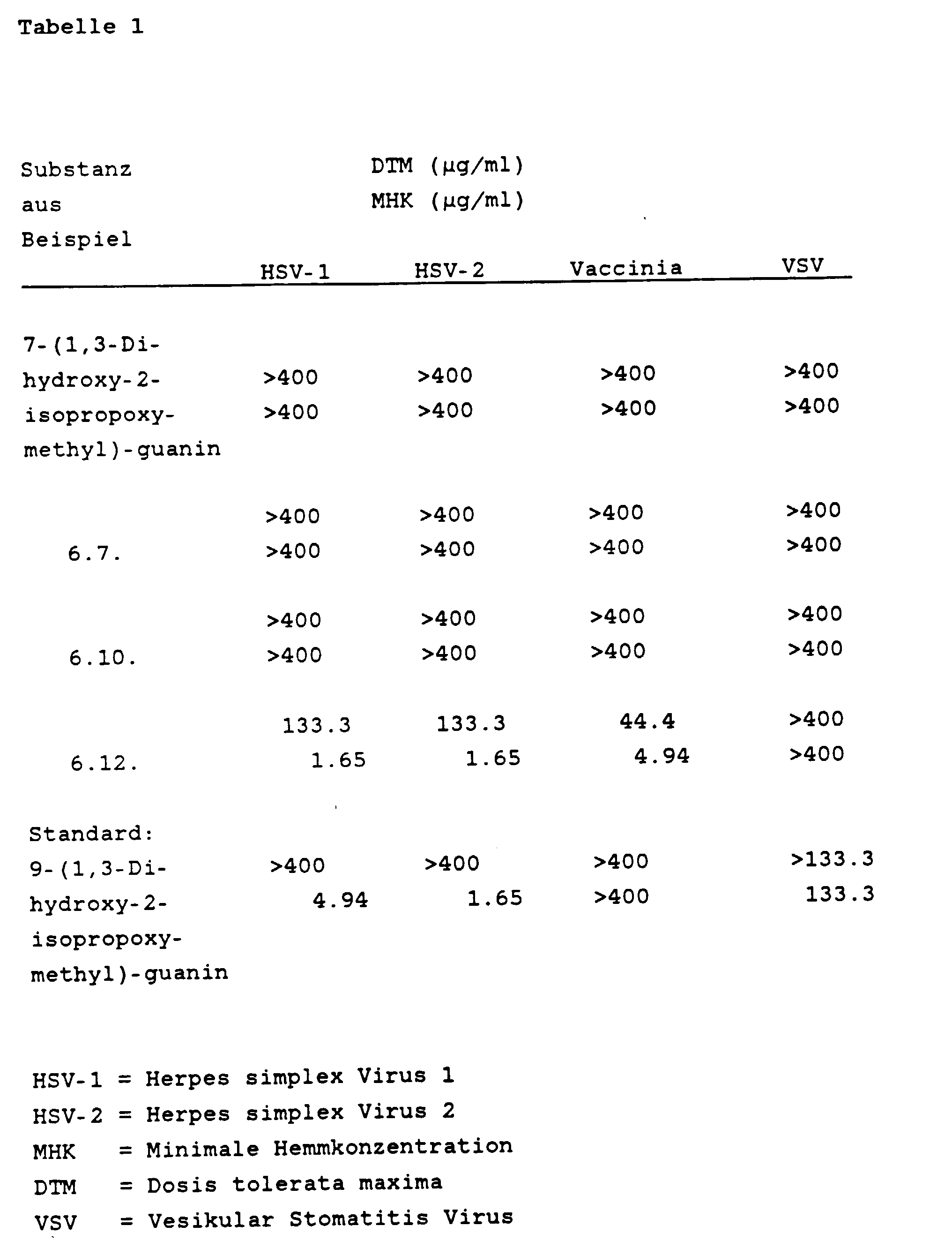

In vivo Versuche und Ergebnisse:

NMRI-Mäuse, spezifisch pathogenfrei, im Gewicht von etwa 15 g wurden intraperitoneal mit Herpes simplex Virus 1 infiziert und anschließend mit den erfindungsgemäßen Verbindungen intraperitoneal oder oral therapiert (s. Tabelle 2 bzw. Tabelle 3). Die Behandlung erfolgte erstmals 3 Stunden nach der Infektion und wurde zweimal täglich über 4 Tage fortgesetzt. Der Behandlungserfolg wurde anhand des Krankheitsverlaufes und der Überlebensrate gegenüber den unbehandelten Infektionskontrollen bestimmt. Letztere erhielten anstelle der erfindungsgemäßen Verbindung physiologische Kochsalzlösung. Die Beobachtungszeit betrug zwei Wochen.

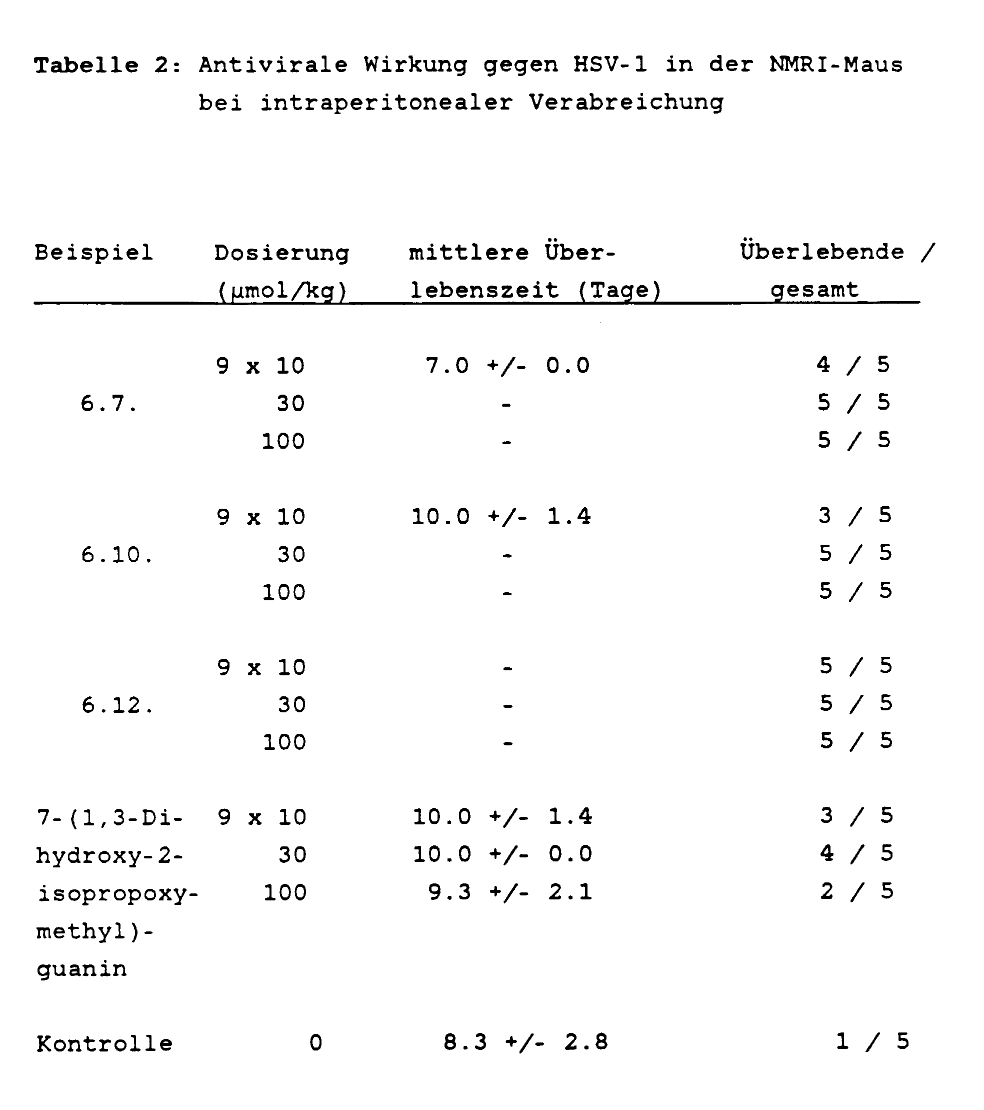

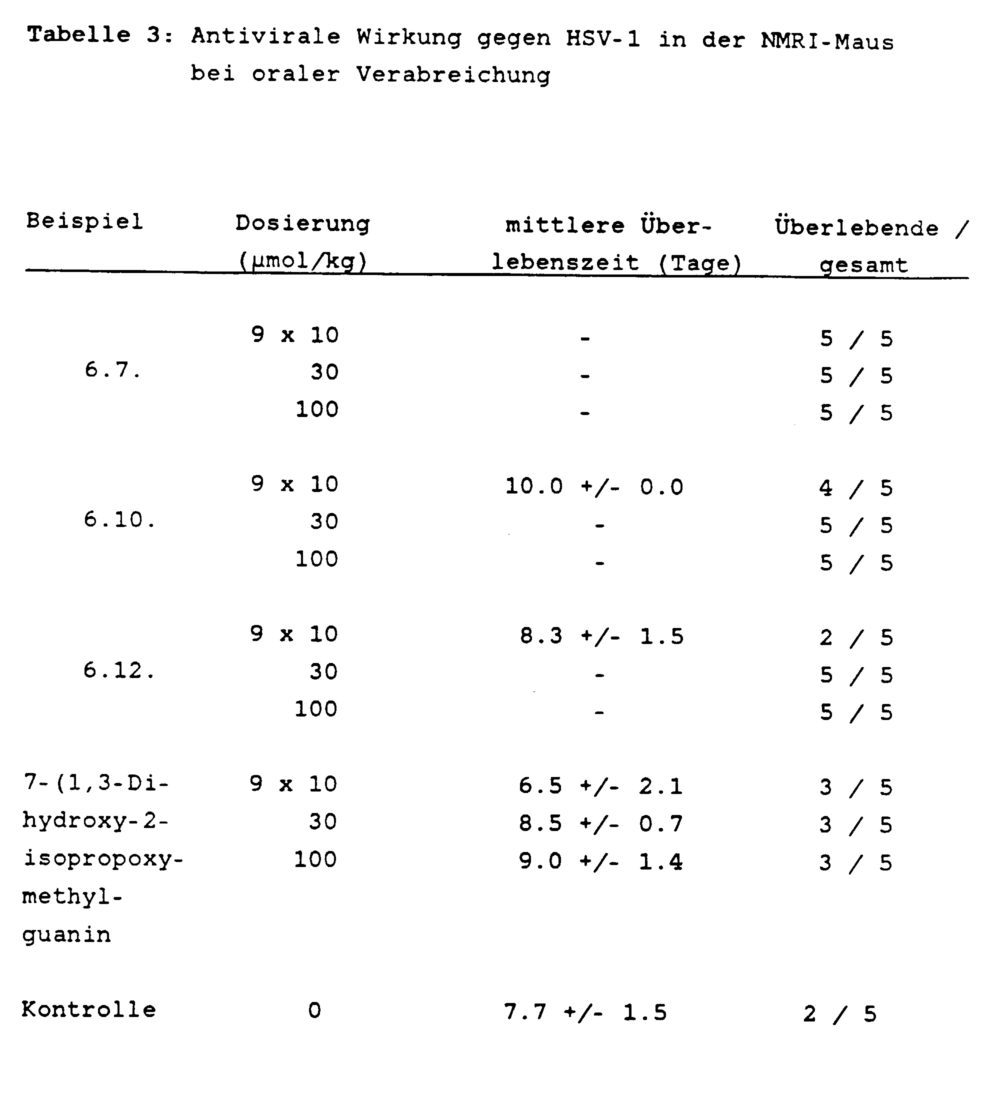

### Beispiele (Verbindungen gemäß Erfindung + Zwischenprodukte):

Verfahren nach 6b):
1. Verbindung der Formel VIII, worin L₂ = Methylthio, R₃ = Isopropoxymethyl, R₄ = Isopropoxy und R₅ = Wasserstoff ist:
   In eine Mischung aus 180 ml Eisessig und 150 ml Essigsäureanhydrid werden unter Rühren und Kühlen auf ca. 30°C langsam 300 ml wasserfreies Dimethylsulfoxid zugetropft. Nach Beendigung der Zugabe wird 30 min. nachgerührt. Sodann werden bei ca. 25°C 52.8 g (0.3 Mol) 2,3-Bis(isopropoxy)-propan-2-ol (hergestellt durch Reaktion von Natriumisopropylat mit 2,3-Epoxypropylisopropylether in Isopropanol) zugetropft. Unter zeitweisem Rühren läßt man die Reaktionsmischung 4 Tage bei Raumtemperatur stehen. Danach wird die Reaktionsmischung in ca. 1 l Eiswasser eingerührt und mehrmals mit Diethylether oder Hexan ausgeschüttelt. Die organische Phase wird mehrmals mit Wasser gewaschen, über Natriumsulfat getrocknet und eingedampft. Der ölige Rückstand wird im Vakuum fraktioniert.
   Die Ausbeute beträgt 53.5 g (75.5% d. Th.) 1,3-Bis(isopropoxy)-2-methylthiomethoxy-propan. Farbloses Öl mit Siedepunkt 68-74°C bei einem Druck von 2 mm Hg.
2. Verbindung der Formel I, worin R₁ = Hydroxy, R₂ = Acetamido, R₃ = Isopropoxymethyl, R₄ = Isopropoxy und R₅ = Wasserstoff ist:
   5.7 g (0.0218 Mol) des Methylthiomethylethers aus Beispiel 1 werden mit 4.9 g wasserfreiem Dimethylsulfoxid und 4.9 g (0.0209 Mol) N2,N9-Diacetylguanin (hergestellt aus Guanin und Essigsäureanhydrid in N-Methylpyrrolidon-(2)) in 20 ml wasserfreiem Dimethylformamid zusammengegeben. Man kühlt auf -20°C und tropft unter Rühren 5.5 g (0.0209 Mol) Zinntetrachlorid in die Suspension. Nach beendeter Zugabe wird die Reaktionsmischung 5 Stunden bei 80°C gerührt. Man läßt abkühlen, versetzt die Reaktionsmischung mit Dichlormethan und Eiswasser, extrahiert mehrmals mit Dichlormethan, schüttelt die vereinigten organischen Phasen erst mit Wasser, dann mit wäßriger Natriumhydrogencarbonatlösung und schließlich mit gesättigter Kochsalzlösung aus. Die organische Phase wird über Natriumsulfat getrocknet, filtriert und eingedampft. Die HPLC-Analyse (RP 18 ((R) LiChrospher 100 RP 18, 5µm, 125-4), Wasser/Methanol 1/1 + 0.1% Trifluoressigsäure, Ammoniumacetat) zeigt ein Verhältnis 7-Isomer/9-Isomer von 47.5/47.7. Die Rohausbeute beträgt 7.5 g (94.4% d. Th.) eines hellen Öls. Die Isolierung des 7-Isomers erfolgt mittels Säulenchromatographie an neutralem Aluminiumoxid mit einem Gemisch von Essigester/Methanol 9/1 und liefert 3.5 g (44% d.Th.) N2-Acetyl-7-[1,3-bis(isopropoxy)-2-propoxymethyl]guanin vom Schmelzpunkt 162-163°C.
   ¹H-NMR (60 MHz, d6-DMSO), ppm: 11.93 (s, breit, 2H), 8.35 (s, 1H), 5.73 (s, 2H), 3.83 (m, 1H), 3.55 - 3.17 (m, 6H), 2.20 (s, 3H), 0.93 (d, 12H).
3.1. Verbindung der Formel I, wobei R₁ = Chlor, R₂ = Acetamido, R₃ = Isopropoxymethyl, R₄ = Isopropoxy und R₅ = Wasserstoff ist:
   6.9 g (0.033 Mol) 2-Acetamido-6-chlor-purin (Herstellung siehe bei 5a.) werden in 28 ml trockenem Xylol mit 28 ml Hexamethyldisilazan (HMDS) und 0.2 g Ammoniumsulfat 3 Stunden unter Argon am Rückfluß gekocht. Danach wird das Lösungsmittel und überschüssiges HMDS abdestilliert, der Rückstand in 85 ml trockenem 1,2-Dichlorethan gelöst und bei -30°C zu einer Lösung von 6.3 g (0.024 Mol) des Methylthiomethylethers aus Beispiel 1 in 85 ml trockenem 1,2-Dichlorethan zugegeben. Sodann fügt man 5 ml (0.026 Mol) Trifluormethansulfonsäuretrimethylsilylester zu und rührt die Mischung 2 Stunden bei -30°C.
   Das Reaktionsprodukt wird in 150 ml Eiswasser eingegossen, filtriert und der Rückstand mit 1,2-Dichlorethan gewaschen. Die wäßrige Phase wird mit 1,2-Dichlorethan ausgeschüttelt; die vereinigten organischen Phasen werden mit Wasser, dann mit verdünnter Natriumhydrogencarbonatlösung ausgeschüttelt, über Natriumsulfat getrocknet und eingeengt. Die HPLC-Analyse (RP 18 (LiChrospher 100 RP 18, 125 x 4), Wasser/Acetonitril 3/1 + 0.1 % TEA) zeigt das Vorhandensein von 73% 2-Acetamido-6-chlor-7-[1,3-bis(isopropoxy)-2-propoxymethyl]-purin neben 2% des entsprechenden 9-Isomeren an.
3.2. Der analoge Umsatz in Acetonitril und mit 3.8 Äquivalenten Zinntetrachlorid ergibt 73 % des 7-Isomeren und 23 % des 9-Isomeren (HPLC-Analyse des Rohproduktes wie in dem vorangegangenen Beispiel).

Verfahren nach 6c):
4.1. Verbindung der Formel VIII, wobei L₂ = Acetoxy, R₃ = Isopropoxymethyl, R₄ = Isopropoxy und R₅ = Wasserstoff ist:
   Zu einer Mischung aus 120 ml Eisessig und 100 ml Essigsäureanhydrid werden unter Rühren 200 ml wasserfreies Dimethylsulfoxid so zugetropft, daß die Temperatur des Gemisches nicht über 35°C steigt. Man rührt weitere 30 Minuten, bevor 35.2 g (0.2 Mol) 1,3-Bis(isopropoxy)-propan-2-ol (wie oben beschrieben dargestellt) zutropft. Nach Beendigung der Zugabe wird die Mischung 7 Stunden lang auf 90 - 100°C erhitzt. Die abgekühlte Reaktionsmischung wird in Wasser gegossen und mehrmals mit Diethylether ausgeschüttelt. Die organische Phase wird dann mit Wasser und anschließend mit wäßriger Hydrogencarbonatlösung gewaschen, über Natriumsulfat getrocknet und eingedampft. Es hinterbleibt ein hellgelbes Öl, das einer fraktionierten Destillation unterworfen wird. Ein Vorlauf mit Siedepunkt 46 -47°C bei einem Druck von 15 mm Hg besteht aus Essigsäurethiomethylmethylester. Das Reaktionsprodukt, 2-Acetoxymethoxy-1,3-bis-(isopropoxy)-propan, siedet bei 87 - 92°C bei einem Druck von 1 mm Hg. Die Ausbeute beträgt 27.3 g (55 % d. Th.).
   ¹H-NMR (60 MHz, CDCl₃), ppm: 5.43 (s, 2H), 4.0 - 3.33 (m, 7H), 2.12 (s, 3H), 1.33 (d, 12H).
   **Auf diese Weise wurden dargestellt:**
   4.2. 2-Acetoxymethoxy-1,3-bis(methoxy)-propan
   4.3. 2-Acetoxymethoxy-1,3-bis(ethoxy)-propan
   4.4. 2-Acetoxymethoxy-1,3-bis(propoxy)-propan
   4.5. 2-Acetoxymethoxy-1,3-bis(benzyloxy)-propan
   4.6. 2-Acetoxymethoxy-1,3-bis(cyclopentyloxy)-propan
   4.7. 2-Acetoxymethoxy-1,3-bis(prop-2-en-1-oxy)-propan
   4.8. 2-Acetoxymethoxy-1-benzyloxy-3-(isopropoxy)-propan
   4.11 2-Acetoxymethoxy-1-benzyloxy-3-pivaloyloxy-propan
   4.12 2-Acetoxymethoxy-1,3-bis(pivaloyloxy)-propan
5. Verbindung der Formel I, wobei R₁ = Chlor, R₂ = Acetamido, R₃ = Isopropoxymethyl, R₄ = Isopropoxy und R₅ = Wasserstoff ist:
5a. 3.17 g (0.015 Mol) 2-Acetamido-6-chlorpurin (hergestellt nach E.M.Acton und R.H.Iwamoto in W.W.Zorbach und R.S.Tipson (Hrsg.) Synthetic Procedures in Nucleic Acid Chemistry, Volume 1, Interscience Publishers, John Wiley & Sons, New York, 1968, S. 25ff) werden in 13 ml wasserfreiem Xylol mit 11.3 ml Hexamethyldisilazan und 100 mg Ammoniumsulfat 3 - 4 Stunden unter Rückfluß in einer Inertgasatmosphäre erhitzt und so in die Bis-Trimethylsilylverbindung überführt. Nach beendeter Reaktion wird das Lösungsmittel und überschüssiges Hexamethyldisilazan im Vakuum abgedampft. Der Rückstand wird in 10 ml wasserfreiem Acetonitril gelöst und unter Rühren zu einer Lösung von 2.8 g (0.01 Mol) 2-Acetoxymethoxy-1,3-bis(isopropoxy)-propan in 70 ml wasserfreiem Acetonitril zugetropft. Sodann gibt man langsam bei -20°C und unter Inertgasatmosphäre 13 g (0.05 Mol) Zinntetrachlorid zu und rührt die Mischung 3 Stunden bei -20°C. Die Reaktionsmischung wird in eine Mischung aus Eiswasser und Dichlormethan eingerührt und filtriert. Die wäßrige Phase wird mehrmals mit Dichlormethan extrahiert und die vereinigten organischen Phasen werden sodann zweimal mit Natriumchloridlösung gewaschen, über Natriumsulfat getrocknet und filtriert und eingedampft. Es bleibt ein heller Sirup zurück, dessen HPLC-Analyse (RP 18 (Nucleosil 5 C18), Wasser/Acetonitril 3/1 + 0.1 % TEA) einen Gehalt von 86 % des 7-Isomeren und 4 % des 9-Isomeren ergibt. Die chromatographische Reinigung an Kieselgel mit Essigester/Methanol 20/1 ergibt 1.8 g (45 % d. Th.) 2-Acetamido-6-chlor-7-[1,3-bis(isopropoxy)-2-propoxymethyl]purin mit dem Schmelzpunkt 73 - 75°C.
   ¹H-NMR (270 MHz, d6-DMSO), ppm: 10.68 (s, 1H), 8.84 (s, 1H), 5.81 (s, 2H), 3.71 (m, 1H), 3.46 - 3.24 (m, 6H), 2.18 (s, 3H), 0.90 (m, 12H).
5b. Reaktionsdurchführung wie bei 5a., mit dem Unterschied, daß das silylierte 2-Acetamido-6-chlorpurin (0.015 Mol) in 10 ml wasserfreiem 1,2-Dichlorethan gelöst zu einer Lösung der Acetoxymethoxyverbindung (0.01 Mol) in 70 ml wasserfreiem 1,2-Dichlorethan zugetropft wird, und man bei -30°C 2.67 g (0.012 Mol) Trifluormethansulfonsäuretrimethylsilylester zugibt und die Reaktionsmischung 2 Stunden bei -30°C rührt. Die HPLC-Analyse des Rohproduktes, durchgeführt wie bei 5a., ergibt ein Isomerenverhältnis von 7-Isomer/9-Isomer wie 90/6. Die chromatographische Reinigung über Kieselgel mit Essigester/Methanol 20/1 liefert 2.15 g (53.8 % d.Th.) weißes Pulver mit Schmelzpunkt 72 - 74°C.

Auf diese Weise wurden dargestellt:
5.1. 2-Acetamido-6-chlor-7-[1,3-bis(ethoxy)-2-propoxymethyl]-purin mit Schmelzpunkt 76 - 78°C
5.2. 2-Acetamido-6-chlor-7-[1,3-bis(propoxy)-2-propoxymethyl]-purin mit Schmelzpunkt 74°C
5.4. 2-Acetamido-6-chlor-7-(1-benzyloxy-3-isopropoxy-2-propoxymethyl)purin als zähes Öl
   (¹H-NMR ( 270 MHz, d6-DMSO), ppm: 10.70 (s, 1H), 8.86 (s, 1H), 7.40 - 7.15 (m, 5H), 5.85 (m, 2H), 4.38 (s, 2H), 3.84 (m, 1H), 3.50 - 3.25 (m, 5H), 2.17 (s, 3H), 0.87 (m, 6H)),
5.5. 2-Acetamido-6-chlor-7-[1,3-bis(methoxy)-2-propoxymethyl]purin mit Schmelzpunkt 83 - 84°C
5.6 2-Acetamido-6-chlor-7-[1,3-bis(prop-2-en-1-oxy)-2-propoxymethyl]purin mit Schmelzpunkt 79°C
5.7 2-Acetamido-6-chlor-7-[1,3-bis(cyclopentyloxy)-2-propoxymethyl]purin als zähes Öl; ¹H-NMR (60 MHz, d6-DMSO), ppm: 10.73 (s,lH), 8.83 (s,lH), 5.83 (s,2H), 3.75 (m,3H), 3.27 (m,4H), 2.18 (s,3H), 1.42 (s, breit, 16H).
5.9 2-Acetamido-6-chlor-7-[1,3-bis(pivaloyloxy)-2-propoxymethyl]purin als glasigen Schaum; ¹H-NMR (210 MHz, d6-DMSO), ppm: 10.72 (s,1H), 8.88 (s,1H), 5.79 (s,2H), 5.04 (m,1H), 4.23 (m,1H), 4.05 (m,1H), 3.63 (d,2H), 2.18 (s,3H), 1.05 (s,9H), 1.01 (s,9H),
5.10 2-Acetamido-6-chlor-7-(1-benzyloxy-3-pivaloyloxy-2-propoxymethyl)-purin als zähes Öl; ¹H-NMR (270 MHz, d6-DMSO), ppm: 10.70 (s,1H), 8.89 (s,1H), 7.35-7.15 (m,5H), 5.85 (s,2H), 4.40 (s,2H), 4.15-3.94 (m,3H), 3.49 (m,2H), 2.18 (s,3H), 0.97 (s,9H).

Verfahren nach 5):
6.1. Verbindung der Formel I, worin R₁ = Mercapto, R₂ = Thioacetamido, R₃ = Isopropoxymethyl, R₄ = Isopropoxy und R₅ = Wasserstoff ist:
   3.8 g (0.01 Mol) der Verbindung aus Beispiel 2. werden in 150 ml trockenem Toluol mit 4.4 g (0.011 Mol) 2,4-Bis-(4-methoxyphenyl)-1,3-dithia-2,4-diphosphetan-2,4-disulfid (Lawesson's Reagenz) 3 Stunden unter Argon bei 80 - 85°C gerührt. Nach Beendigung der Reaktion läßt man abkühlen, saugt vom Niederschlag ab, wäscht den Rückstand mit Toluol und dampft das Filtrat ein. Es bleibt ein gelber Sirup zurück, der chromatographisch über Kieselgel mit Essigester/Methanol 20/1 gereinigt wird. Auf diese Weise erhält man 2 g (48.6 % d. Th.) 2-Thioacetyl-7-(1,3-bis(isopropoxy)-2-propoxymethyl)thioguanin. Das gelbliche Pulver zersetzt sich bei 220°C.
6.2. Verbindung der Formel I, worin R₁ = Mercapto, R₂ = Amino, R₃ = Isopropoxymethyl, R₄ = Isopropoxy und R₅ = Wasserstoff ist:
   1.6 g (0.00472 Mol) 7-(1,3-Bis(isopropoxy)-2-propoxymethyl)-guanin werden in einer Mischung aus 50 ml trockenem Toluol und 10 ml trockenem Pyridin mit 1.01 g (0.0025 Mol) Lawesson's Reagenz unter Argon 6 Stunden unter Rückfluß erhitzt. Das Rohprodukt der Reaktion wird säulenchromatographisch an Kieselgel mit einem Gemisch von Dichlormethan/Methanol 5/1 gereinigt. Man erhält 1.2 g (71.6 % d. Th.) eines schwach gelben Pulvers mit Schmelzpunkt 232 - 236°C.
   ¹H- NMR (60 MHz, d6-DMSO), ppm: 12.05 (s, 1H), 8.37 (s, 1H), 6.55 (s, 2H), 6.10 (s, 2H), 3.93-3.22 (m, 7H), 0.97 (d, 12H).
   Die Verbindung 6.2. kann auch hergestellt werden, indem man 0.413 g (0.001 Mol) der Verbindung des Beispiels 6.1. mit 4 ml 40 %iger wäßriger Methylamin-Lösung und 4 ml Methanol 3 Stunden zum Rückfluß erhitzt. Ausbeute: 0.25 g (70.4% d. Th.).
6.3. Verbindung der Formel I, worin R₁ = Methoxy, R₂ = Acetamido, R₃ = Isopropoxymethyl, R₄ = Isopropoxy und R₅ = Wasserstoff ist:
   1.4 g (0.0035 Mol) der Verbindung aus Beispiel 5. werden mit 13 mg (0.2 mMol) Kaliumcyanid in 14 ml Methanol gelöst und 24 Stunden bei Raumtemperatur gerührt. Danach wird mit 20 ml Methanol verdünnt und mit Serdolit Blue (R) (Serva) (OH- Form) und Amberlyst 15 (R) (Fluka) (H+ Form) jeweils 5 Minuten behandelt, vom Ionenaustauscher abfiltriert und das Filtrat eingedampft. Der hinterbleibende Sirup kristallisiert auf Zugabe von Diisopropylether. Man erhält 1.05 g (75.8 % d. Th.) 2-Acetamido-6-methoxy-7-(1,3-bis(isopropoxy)-2-propoxymethyl)-purin vom Schmelzpunkt 67 - 68°C.
   ¹H-NMR (60 MHz, d6-DMSO), ppm: 10.73 (s, 1H), 8.87 (s, 1H), 5.83 (s, 2H), 3.80 - 3.15 (m, 10H), 2.17 (s, 3H), 0.88 (d, 12H).
6.4.1. Verbindungen der Formel I, worin R₁ = Amino, R₂ = Acetamido, R₃ = Isopropoxymethyl, R₄ = Isopropoxy und R₅ = Wasserstoff
   und
6.4.2. R₁ = Amino, R₂ = Amino, R₃ = Isopropoxymethyl, R₄ = Isopropoxy und R₅ = Wasserstoff ist:
6.4.1. 10 g (0.025 Mol) der Verbindung aus Beispiel 2. werden in 100 ml Methanol mit ca. 150 ml flüssigem Ammoniak versetzt und 20 Stunden im Autoklaven bei einem Druck von 5 bar auf 80°C erhitzt. Danach wird das Reaktionsgemisch vollständig eingedampft und säulenchromatographisch (Kieselgel, Dichlormethan/Methanol 9/1) gereinigt. Als erste Fraktion erhält man 0.26 g (2.7 % d. Th.) 2-Acetamido-6-amino-7-[1,3-bis(isopropoxy)-2-propoxymethyl]purin mit dem Schmelzpunkt 136 - 137°C.
   ¹H-NMR (60 MHz, d6-DMSO), ppm: 9.72 (s, 1H), 8.30 (s,lH), 6.73 (s, 2H), 5.73 (s, 2H), 3.83 - 3.20 (m, 7H), 2.20 (s, 3H), 0.97 (d, 12H).
6.4.2. Die zweite Fraktion liefert 4.6 g (54.4 % d. Th.) 2,6-Diamino-7-[1,3-bis(isopropoxy)-2-propoxymethyl]purin mit dem Schmelzpunkt 228 - 229°C.
   ¹H-NMR (60 MHz, d6-DMSO), ppm: 8.10 (s, 1H), 6.52 (s,2H), 5.85 (s, 2H), 5.67 (s, 2H), 3.83 - 3.22 (m, 7H), 1.00 (d, 12H).
6.5.1. Verbindungen der Formel I, worin R₁ = Methylamino, R₂ = Acetamido, R₃ = Isopropoxymethyl, R₄ = Isopropoxy und R₅ = Wasserstoff
   und
6.5.2. R₁ = Methylamino, R₂ = Amino, R₃ = Isopropoxymethyl, R₄ = Isopropoxy, R₅ = Wasserstoff ist:
6.5.1. 1.4 g (3.5 mMol) der Verbindung aus Beispiel 2 werden mit 7 ml 40 %iger wäßriger Methylamin-Lösung und 14 ml Methanol 2 Stunden unter Rückfluß erhitzt. Danach wird die Reaktionslösung vollständig eingedampft und der Rückstand säulenchromatographisch an Kieselgel mit einem Gemisch von Dichlormethan/Methanol 10/1 aufgetrennt. Die erste Fraktion besteht aus 0.45 g (32.6 % d. Th.) 2-Acetamido-6-methylamino-7-[1,3-bis(isopropoxy)-2-propoxymethyl]purin mit Schmelzpunkt 159°C.
   ¹H-NMR (60 MHz, d6-DMSO), ppm: 9.75 (s, 1H), 8.28 (s, 1H), 6.67 (q, 1H), 5.75 (s, 2H), 3.77 - 3.22 (m, 7H), 3.00 (d, 3H), 2.27 (s, 3H), 0.97 (d, 12H).
6.5.2. Die zweite Fraktion besteht aus 0.45 g (36.5 % d. Th.) 2-Amino-6-methylamino-7-[1,3-bis-(isopropoxy)-2-propoxy-methyl]purin vom Schmelzpunkt 103 - 104°C.
   ¹H-NMR (270 MHz, d6-DMSO), ppm: 7.99 (s, 1H), 6.29 (q, 1H), 5,65 (s, 2H), 5.63 (s, 2H), 3.65 (m, 1H), 3.45 (m, 2H), 3.36 (m, 4H), 2.92 (d, 3H), 1.00 (q, 12H).
   Verwendet man einen größeren Überschuß an Methylamin-Lösung und verlängert man die Reaktionszeit, so isoliert man ausschließlich das entblockierte Produkt 6.4.2. in 92 %iger Ausbeute.
6.6. Verbindung der Formel I, worin R₁ = Wasserstoff, R₂ = Acetamido, R₃ = Isopropoxymethyl, R₄ = Isopropoxy und R₅ = Wasserstoff ist:
   16 g (0.04 Mol) der Verbindung aus Beispiel 5 werden in 350 ml Methanol mit 3.5 g Palladium auf Kohle (10 %) und 11.06 ml (0.08 Mol) Triethylamin in der Schüttelente bei Raumtemperatur mit Wasserstoff erschöpfend hydrogenolysiert. Nach Beendigung der Wasserstoffaufnahme wird vom Katalysator abgesaugt und die methanolische Phase eingedampft. Der Rückstand wird in Essigester angerührt, der Niederschlag von Triethylamin-Hydrochlorid wird abgetrennt und die Essigesterlösung vollständig eingeengt. Der kristalline Rückstand wird säulenchromatographisch über Kieselgel mit Essigester/Methanol 9/1 als Elutionsmittel gereinigt. Man erhält 14 g (95.9 % d. Th.) 2-Acetamido-7-[1,3-bis(isopropoxy)-2-propoxymethyl]-purin vom Schmelzpunkt 94 - 96°C.
   ¹H-NMR (270 MHz, d6-DMSO), ppm: 10.43 (s, 1H), 9.02 (s,1H), 8.71 (s, 1H), 5.79 (s, 2H), 3.70 (m, 1H), 3.41 (m, 2H), 3.32 (m, 4H), 2.19 (s, 3H), 0.93 (m, 12H).
6.7. Verbindung der Formel I, worin R₁ = Wasserstoff, R₂ = Amino, R₃ = Isopropoxymethyl, R₄ = Isopropoxy und R₅ = Wasserstoff ist:
   0.178 g (0.5 mMol) der Verbindung des Beispiels 6.2. werden in 20 ml absolutem Ethanol mit 1.0 g mit absolutem Ethanol gewaschenem Raney-Nickel versetzt und 1.5 Stunden zum Rückfluß erhitzt. Danach wird die Reaktionsmischung abgekühlt, vom Raney-Nickel abgesaugt und die ethanolische Lösung vollständig eingeengt. Der Rückstand wird säulenchromatographisch über Kieselgel mit Essigester/Methanol 20/1 als Elutionsmittel gereinigt. Man erhält 0.1 g (61.7 % d. Th.) 2-Amino-7-[1,3-bis(isopropoxy)-2-propoxymethyl]purin als weiße Flitter vom Schmelzpunkt 153 - 154°C.
   ¹H-NMR (270 MHz, d6-DMSO), ppm: 8.65 (s, 1H), 8.38 (s, 1H), 6.22 (s, 2H), 5.67 (s, 2H), 3.65 (m, 1H), 3.42 (m, 2H), 3.32 (m, 4H), 1.00 (m, 12H).
   Die Verbindung des Beispiels 6.7. kann auch aus der Verbindung des Beispiels 6.6. durch Reaktion mit wäßriger Methylamin-Lösung in Methanol hergestellt werden. 1.1 g der Verbindung des Beispiels 6.6. liefern 0.8 g (82.2 % d. Th.) der Verbindung des Beispiels 6.7.
6.9. Verbindung der Formel I, worin R₁ = Wasserstoff, R₂ = Acetamido, R₃ = Benzyloxymethyl, R₄ = Isopropoxy und R₅ = Wasserstoff ist:
   4.47 g (0.01 Mol) der Verbindung des Beispiels 5.4. werden wie in Beispiel 6.6. der Hydrogenolyse unterworfen und liefern 3 g (72.6 % d.Th.) 2-Acetamido-7-(1-benzyloxy-3-isopropoxy-2-propoxymethyl)-purin als Öl.
   ¹H-NMR (270 MHz, d6-DMSO), ppm: 10.43 (s, 1H), 9.03 (s, 1H), 8.72 (s, 1H), 7.36 - 7.15 (m, 5H), 5.82 (m, 2H), 4.39 (s, 2H), 3.83 (s, 1H), 3.50 - 3.25 (m, 5H), 2.18 (s, 3H), 0.90 (m, 6H).
6.10. Verbindung der Formel I, worin R₁ = Wasserstoff, R₂ = Amino, R₃ = Hydroxymethyl, R₄ = Isopropoxy und R₅ = Wasserstoff ist:
   1.6 g (4.95 mMol) der Verbindung des Beispiels 8.3. werden in 50 ml Methanol mit 50 ml 40 %iger wäßriger Methylaminlösung 1 Stunde unter Rückfluß erhitzt. Nach dem Abdestillieren des Lösungsmittels hinterbleibt ein Öl, welches mit Aceton versetzt wird und nach einiger Zeit kristallisiert. Man erhält 0.7 g (50.3 % d. Th.) 2-Amino-7-(1-hydroxy-3-isopropoxy-2-propoxymethyl)-purin mit dem Schmelzpunkt 125 - 130°C.
   ¹H-NMR (270 MHz, d6-DMSO), ppm: 8.66 (s,1H), 8.37 (s, 1H), 6.23 (s, 2H), 5.67 (m, 2H), 4.70 (t, 1H), 3.55 (m, 1H), 3.36 - 3.20 (m, 5H), 0.95 (m, 6H).
6.12. Verbindung der Formel I, worin R₁ = Wasserstoff, R₂ = Amino, R₃ = Hydroxymethyl, R₄ = Hydroxy und R₅ = Wasserstoff ist:
   1.6 g (0.0057 Mol) der Verbindung des Beispiels 8.2. werden mit 10 ml Methanol, 10 ml 40 %iger wäßriger Methylamin-Lösung und 5 ml Wasser 2 Stunden zum Rückfluß erhitzt. Die Aufarbeitung liefert 1 g (73.4 % d. Th.) 2-Amino-7-(1,3-dihydroxy-2-propoxymethyl)-purin vom Schmelzpunkt 176 - 177°C.
   ¹H-NMR (270 MHz, d6-DMSO), ppm: 8.68 (s, 1H), 8.38 (s, 1H), 6.23 (s, 2H), 5.69 (s, 2H), 4.62 (t, 2H), 3.38 (m, 5H).
6.13.1. Verbindung der Formel I, worin R₁ = Hydroxy, R₂ = Hydroxy, R₃ = Isopropoxymethyl, R₄ = Isopropoxy und R₅ = Wasserstoff ist
   und
6.13.2. Verbindung der Formel I, worin R₁ = Amino, R₂ = Hydroxy, R₃ = Isopropoxymethyl, R₄ = Isopropoxy und R₅ = Wasserstoff ist.
6.13.1. 1.4 g (0.0041 Mol) der Verbindung des Beispiels 6.4.2. werden in einer Mischung aus 45 ml Tetrahydrofuran und 30 ml Wasser gelöst. Man fügt 1.8 g (0.027 Mol) Natriumnitrit und 24 ml Eisessig zu, rührt 90 Minuten bei 50°C, fügt nochmals 1.8 g Natriumnitrit und 9 ml Eisessig zu, dampft darauf das Reaktionsgemisch vollständig ein, versetzt mit wenig Wasser und neutralisiert mit konzentriertem Ammoniak. Es scheidet sich ein Öl ab, das nach einiger Zeit fest wird. Man saugt den Niederschlag ab, kristallisiert ihn aus Isopropanol um und erhält 0.3 g (21.3 % d. Th.) 7-[1,3-Bis(isopropoxy)-2-propoxymethyl]-xanthin vom Schmelzpunkt 200 - 201°C.
   ¹H-NMR (270 MHz, d6-DMSO), ppm: 11.60 (s, 1H), 10.89 (s,1H), 8.13 (s, 1H), 5.63 (s, 2H), 3.83 (m, 1H), 3.46 (m, 2H), 3.30 (m, 4H), 1.01 (m, 12H).
6.13.2. Die wäßrigen Mutterlaugen werden eingedampft, mit Dichlormethan und wenig Wasser aufgenommen und dreimal mit 100 ml Dichlormethan ausgeschüttelt. Die organische Phase wird getrocknet (Natriumsulfat) und eingedampft. Der so erhaltene Sirup wird chromatographisch (Kieselgel, Dichlormethan/Methanol 9/1) gereinigt. Nach Umkristallisation aus Wasser werden 60 mg (4.3 % d. Th.) 6-Amino-2-hydroxy-7-[1,3-bis(isopropoxy)-2-propoxymethyl]purin <7-[1,3-Bis(isopropoxy)-2-propoxymethyl]isoguanin> vom Schmelzpunkt 213°C erhalten.
   ¹H-NMR (270 MHz, d6-DMSO), ppm: 11.15 (s, 1H), 8.02 (s, 1H), 6.88 (s, 2H), 5.64 (s, 2H), 3.70 (m, 1H), 3.47 (m, 2H), 3.38 (m, 4H), 1.01 (d, 12H).
6.14. Verbindung der Formel I, worin R₁= Wasserstoff, R₂= Acetamido, R₃= Methoxymethyl, R₄= Methoxy und R₅= Wasserstoff ist:
   1.6 g (4.7 mMol) der Verbindung des Beispiels 5.5. werden wie in Beispiel 6.6. der Hydrogenolyse unterworfen und liefern nach Chromatographie an Kieselgel (Essigsäureethylester/Methanol 5/1) 1.25 g (86 % d.Th.) 2-Acetamido-7- 1,3-bis(methoxy)-2-propoxymethyl purin mit dem Schmelzpunkt 101-102°C.
6.15. Verbindung der Formel I, wobei R₁= Wasserstoff, R₂= Acetamido, R₃= Ethoxymethyl, R₄= Ethoxy und R₅= Wasserstoff ist:
   3.7 g (0.01 Mol) der Verbindung des Beispiels 5.1. werden wie in Beispiel 6.6. der Hydrogenolyse unterworfen und liefern nach chromatographischer Reinigung an Kieselgel (Essigsäureethylester/Methanol 9/1) 2.9 g (86 % d.Th.) 2-Acetamido-7-[1,3-bis(ethoxy 2-propoxymethyl]-purin mit dem Schmelzpunkt 117-118°C
6.16. Verbindung der Formel I, wobei R₁= Wasserstoff, R₂= Amino, R₃= Prop-2-en-1-oxymethyl, R₄= Prop-2-enoxy und R₅= Wasserstoff ist:
   4.35 g (11 mMol) Verbindung des Beispiels 5.6. werden den mit 3.84 g Zinkstaub in 60 ml Wasser zum Rückfluß erhitzt. Sodann werden über einen Zeitraum von 2 Stunden 1.7 ml konzentrierter Ammoniak zugetropft. Die abgekühlte Suspension wird mit 50 ml Methanol versetzt und abgesaugt; der Rückstand wird mit Methanol gewaschen. Die vereinigten Filtrate werden eingeengt und über Kieselgel mit Essigsäureethylester/Methanol 9/1 chromatographiert. Man erhält 2.9 g (82.6 % d.Th.) 2-Amino-7-[1,3-bis-(prop-2-en-1-oxy)-2-propoxymethyl]purin mit dem Schmelzpunkt 140-143°C
6.17 Verbindung der Formel I, wobei R₁= Wasserstoff, R₂= Acetamido, R₃= Cyclopentyloxymethyl, R₄= Cyclopentyloxy und R₅= Wasserstoff ist:
   1.1 g (2.44 mMol) der Verbindung des Beispiels 5.7. werden wie in Beispiel 6.6. der Hydrogenolyse unterworfen und liefern nach chromatographischer Reinigung (Kieselgel, Essigsäureethylester/Methanol 9/1) 0.8 g (78.6 % d.Th.) 2-Acetamido-7-[1,3-bis-(cyclopentyloxy)-2-propoxymethyl]purin mit dem Schmelzpunkt 98°C
6.19. Verbindung der Formel I, wobei R₁= Wasserstoff, R₂= Acetamido, R₃= Benzyloxymethyl, R₄= Pivaloyloxy und R₅= Wasserstoff ist:
   20.5 g (41.9 mMol) der Verbindung des Beispiels 5.10. werden wie in Beispiel 6.6. der Hydrogenolyse unterworfen und liefern nach Chromatographie an Kieselgel (Essigsäureethylester/Methanol 9/1) 17 g (89.2 % d. Th.) 2-Acetamido-7-(1-benzyloxy-3-pivaloyloxy-2-propoxymethyl)purin mit dem Schmelzpunkt 76-77°C. ¹H-NMR (270 MHz, d₆-DMSO), δ ppm : 10.45 (s,1H), 9.05 (s,1H), 8.77 (s,1H), 7.35-7.18 (m,5H), 5.83 (s,2H), 4.40 (2,2H), 4.13 (m,1H), 3.99 (m,2H), 3.49 (m2H), 2.19 (s,3H), 0.98 (s,9H).
6.20. Verbindung der Formel I, worin R₁= Wasserstoff, R₂= Amino, R₃= Methoxymethyl, R₄= Methoxy und R₅= Wasserstoff ist:
   0.77 g (2.25 mMol) der Verbindung des Beispiels 6.14. werden wie in Beispiel 6.10. mit Methylaminlösung behandelt und liefern 0.54 g (89.9 % d.Th.) 2-Amino-7-[1,3-bis(methoxy)-2-propoxymethyl]purin mit dem Schmelzpunkt 148°C.
6.21. Verbindung der Formel I, worin R₁= Wasserstoff, R₂= Amino, R₃= Ethoxymethyl, R₄= Ethoxy und R₅= Wasserstoff ist:
   1.35 g (4 mMol) der Verbindung des Beispiels 6.15. werden wie in Beispiel 6.10. mit Methylaminlösung behandelt und liefern 0.8 g (67.8 % d. Th.) 2-Amino-7-[1,3-bis-(ethoxy)-2-propoxymethyl]purin mit dem Schmelzpunkt 151°C.
6.22 Verbindung der Formel I, worin R₁= Wasserstoff, R₂= Amino, R₃= Cyclopentyloxymethyl, R₄= Cyclopentyloxy und R₅= Wasserstoff ist:
   0.5 g (1.2 mMol) der Verbindung des Beispiels 6.17. werden wie in Beispiel 6.10. mit Methylaminlösung behandelt und liefern 0.3 g (66.7 % d.Th.) 2-Amino-7-[1,3-bis-(cyclopentyloxy)-2-propoxymethyl]-purin mit dem Schmelzpunkt 158°C.
6.24. Verbindung der Formel I, worin R₁= Wasserstoff, R₂= Amino, R₃= Benzyloxymethyl, R₄= Hydroxy und R₅= Wasserstoff ist:
   0.66 g (1.45 mMol) der Verbindung des Beispiels 6.19. werden wie in Beispiel 6.10. mit Methylaminlösung behandelt und liefern 0.25 g (52.4 % d.Th.) 2-Amino-7-(1-benzyloxy-3-hydroxy-2-propoxymethyl)-purin mit dem Schmelzpunkt 122°C; ¹H-NMR (270 MHz, d₆- DMSO), δ ppm : 8.68 (s,1H), 8.39 (s,1H), 7.36-7.18 (m,5H), 6.25 (s,2H), 5.71 (s,2H), 4.73 (t,1H), 4.38 (s,2H), 3.68 (m,1H), 3.50-3.31 (m,4H).
6.25. Verbindung der Formel I, worin R₁= Wasserstoff, R₂= Acetamido, R₃= Benzyloxymethyl, R₄= Hydroxy und R₅= Wasserstoff ist:
   0.5 g (1.1 mMol) der Verbindung des Beispiels 6.19. werden in 10 ml Methanol gelöst, mit 10 ml konzentriertem wäßrigen Ammoniak versetzt und 24 Stunden bei Raumtemperatur gerührt. Die Aufarbeitung liefert 0.25 g (61.3 % d. Th.) 2-Acetamido-7-(1-benzyloxy-3-hydroxy-2-propoxymethyl)-purin mit dem Schmelzpunkt 149°C; ¹H-NMR (60 MHz, d₆-DMSO), δ ppm : 10.47 (s,1H), 9.10 (s,1H), 8.78 (s,1H), 7.30 (m,5H), 5.87 (s,2H), 4.73 (t,1H), 4.40 (s,2H), 3.80-3.30 (m,5H), 2.17 (s,3H).
6.26 Verbindung der Formel I, worin R₁= Wasserstoff, R₂= Acetamido, R₃= Acetoxymethyl, R₄= Acetoxy und R₅= Wasserstoff ist:
   0.24 g (1 mMol) der Verbindung des Beispiels 6.12. werden mit 10 ml Essigsäureanhydrid und 30 mg N,N-Dimethylaminopyridin versetzt und 18 Stunden bei Raumtemperatur gerührt. Nach Neutralisation der Reaktionsmischung und chromatographischer Reinigung des Rohproduktes über Kieselgel mit Essigsäureethylester/Methanol 9/1 werden 0.2 g (61.9 % d. Th.) 2-Acetamido-7-[1,3-bis(acetoxy)-2-propoxymethyl]purin mit dem Schmelzpunkt 141°C erhalten. ¹H-NMR (270 MHz, d₆-DMSO), δ ppm : 10.46 (s,1H), 9.04 (s,1H), 8.77 (s,1H), 5.82 (s,2H), 4.16-4.09 (m,2H), 4.05-3.95 (m,3H), 2.19 (s,1H), 1.70 (s,6H).

Kombination der Verfahren nach 1), 2) und 4) mit dem Verfahren nach 5):
7.1. Verbindung der Formel I, worin R₁ = Isopropoxy, R₂ = Amino, R₃ = Isopropoxymethyl, R₄ = Isopropoxy und R₅ = Wasserstoff ist:
   2 g (0.005 Mol) der Verbindung aus Beispiel 5 werden in 25 ml wasserfreiem Isopropanol gelöst, mit einer Lösung von 0.345 g (0.015 Mol) Natrium in wasserfreiem Isopropanol versetzt und 2 Stunden zum Rückfluß erhitzt. Die abgekühlte Suspension wird mit Eiswasser versetzt und mit 2 N Essigsäure neutralisiert. Der Niederschlag wird abgesaugt, mit Wasser gewaschen und getrocknet. Man erhält 1.5 g (78.6 % d. Th.) 2-Amino-6-isopropoxy-7-[1,3-bis-(isopropoxy)-2-propoxymethyl]-purin mit dem Schmelzpunkt 85 - 87°C.
   ¹H-NMR (60 MHz, d6-DMSO), ppm: 8.22 (s, 1H), 6.08 (s, 2H), 5.62 (s, 2H), 5.50 (m, 1H), 3.87 - 3.17 (m, 7H), 1.35 (d, 6H), 0.97 (d, 12H).
7.2. Verbindung der Formel I, worin R₁ = Methoxy, R₂ = Amino, R₃ = Isopropoxymethyl, R₄ = Isopropoxy und R₅ = Wasserstoff ist:
   2 g (0.005 Mol) der Verbindung des Beispiels 5. werden in 20 ml Methanol gelöst und zu einer Lösung von Natriummethanolat in Methanol (0.35 g Natrium, 20 ml Methanol) gegeben. Die Mischung wird 2 Stunden unter Rückfluß gekocht. Ausgefallenes Natriumchlorid wird abgesaugt, und die methanolische Lösung wird vollständig eingeengt. Der Rückstand wird in wenig Wasser gelöst und mit Essigsäure neutralisiert. Das ausgefallene Produkt wird abgesaugt, mit Wasser gewaschen und getrocknet. Man erhält 1.5 g (84.9 % d.Th.) 2-Amino-6-methoxy-7-[1,3-bis(isopropoxy)-2-propoxymethyl]-purin mit dem Schmelzpunkt 111°C.

Verfahren nach 1), 2) und 4):
8.1. Verbindung der Formel I, worin R₁ = Hydroxy, R₂ = Acetamido, R₃ = Hydroxymethyl, R₄ = Hydroxy und R₅ = Wasserstoff ist:
   3.81 g (0.01 Mol) der Verbindung des Beispiels 2 werden in 150 ml wasserfreiem Dichlormethan gelöst und unter Rühren in einer Argonatmosphäre auf -60°C gekühlt. Sodann fügt man langsam 60 ml (0.06 Mol) einer 1 molaren Lösung von Bortrichlorid in n-Hexan oder Dichlormethan zu, läßt die Temperatur der Reaktionsmischung langsam auf -40°C bis -20°C ansteigen, rührt 3 Stunden bei dieser Temperatur und dann noch 1 Stunde bei -10°C. Man kühlt erneut auf -60°C, tropft langsam 60 ml Methanol und 60 ml Dichlormethan zu und erhält eine Lösung, die mit 37 ml Triethylamin versetzt wird. Es wird noch 30 Minuten bei Raumtemperatur nachgerührt bevor die Reaktionsmischung vollständig eingedampft wird. Chromatographie an Kieselgel mit einem Gemisch von Dichlormethan/Methanol 3/1 liefert 1.32 g (44.4 % d. Th.) 2-Acetyl-7-(1,3-dihydroxy-2-propoxymethyl)-guanin vom Schmelzpunkt 155 - 158°C (Zersetzung).
   ¹H-NMR (270 MHz, d6-DMSO), ppm: 12.15 (s, 1H), 11.61 (s, 1H), 8.37 (s, 1H), 5.77 (s, 2H), 4.61 (t, 2H), 3.62 (m, 1H), 3.35 (m, 4H), 2.17 (s, 3H).
8.2. Verbindung der Formel I, worin R₁ = Wasserstoff, R₂ = Acetamido, R₃ = Hydroxymethyl, R₄ = Hydroxy und R₅ = Wasserstoff ist:
   Nach derselben Methode wurden 3.65 g (0.01 Mol) der Verbindung des Beispiels 6.6. mit 0.05 Mol Bortrichlorid umgesetzt und lieferten nach Kristallisation aus Methanol 2.2 g (78.3 % d. Th.) 2-Acetamido-7-(1,3-dihydroxy-2-propoxymethyl)-purin vom Schmelzpunkt 214 - 215°C.
   ¹H-NMR (270 MHz, d6-DMSO), ppm: 10.43 (s, 1H), 9.07 (s, 1H), 8.72 (s, 1H), 5.81 (s, 2H), 4.65 (t, 2H), 3.55 - 3.28 (m, 5H), 2.20 (s, 3H).
8.3. Verbindung der Formel I, worin R₁ = Wasserstoff, R₂ = Acetamido, R₃ = Hydroxymethyl, R₄ = Isopropoxy und R₅ = Wasserstoff ist:
   3 g der Verbindung des Beispiels 6.9. werden in 75 ml Methanol mit 4 g Ammoniumformiat und 1 g Palladium/Kohle (10 %) 8 Stunden unter Rückfluß erhitzt. Die Reaktionsmischung wird filtriert, eingeengt, mit Aceton versetzt und bis zur Kristallisation gerührt. Man erhält 0.9 g (57.4 % d.Th.) 2-Acetamido-7-(1-hydroxy-3-isopropoxy-2-propoxymethyl)-purin mit dem Schmelzpunkt 170°C.
   ¹H-NMR (270 MHz, d6-DMSO), ppm: 10.43 (s, 1H), 9.04 (s, 1H), 8.72 (s, 1H), 5.80 (m, 2H), 4.73 (t, 1H), 3.60 (m, 1H), 3.46 - 3.20 (m, 5H), 2.17 (s, 3H), 0.90 (m, 6H).

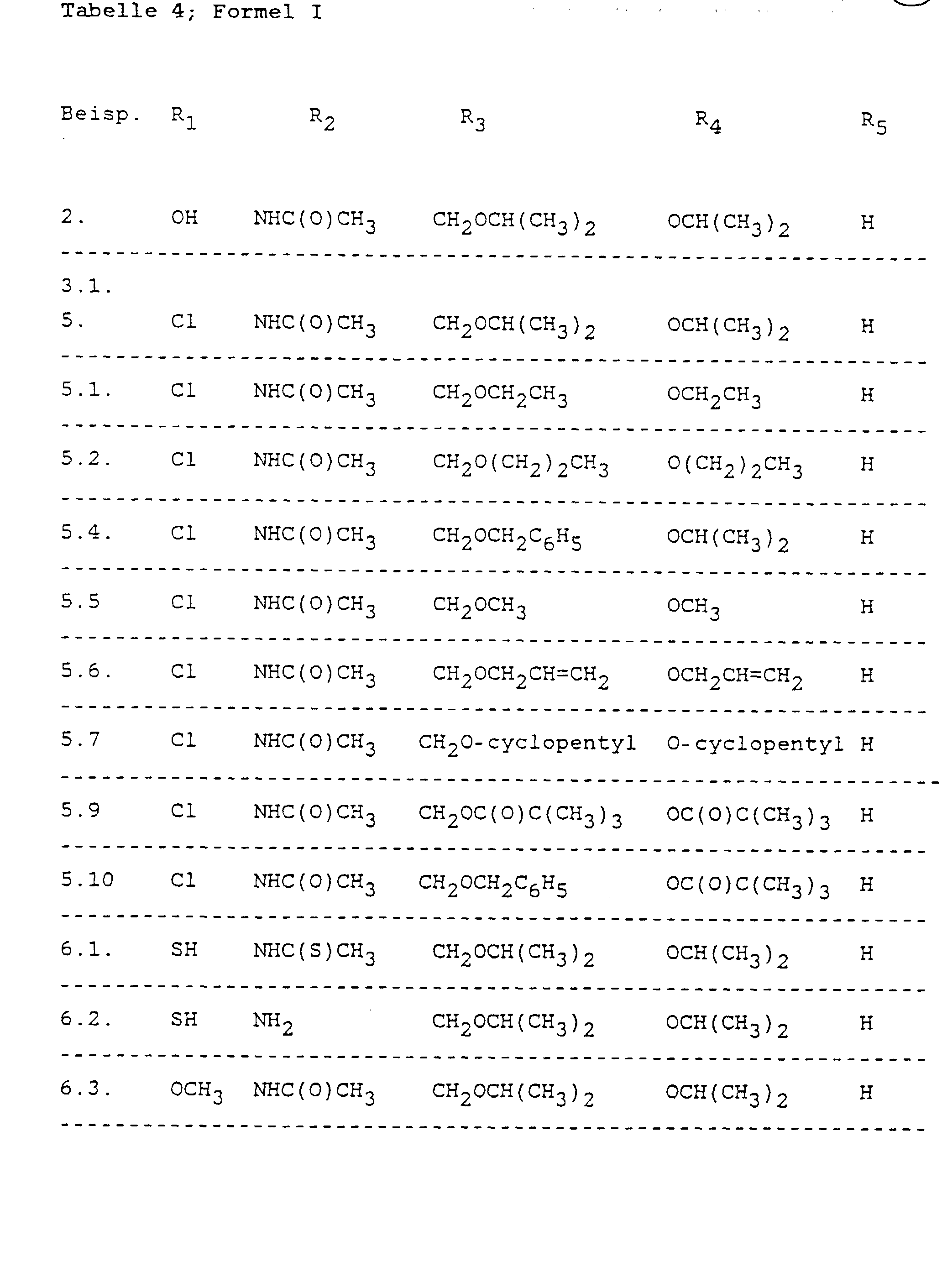

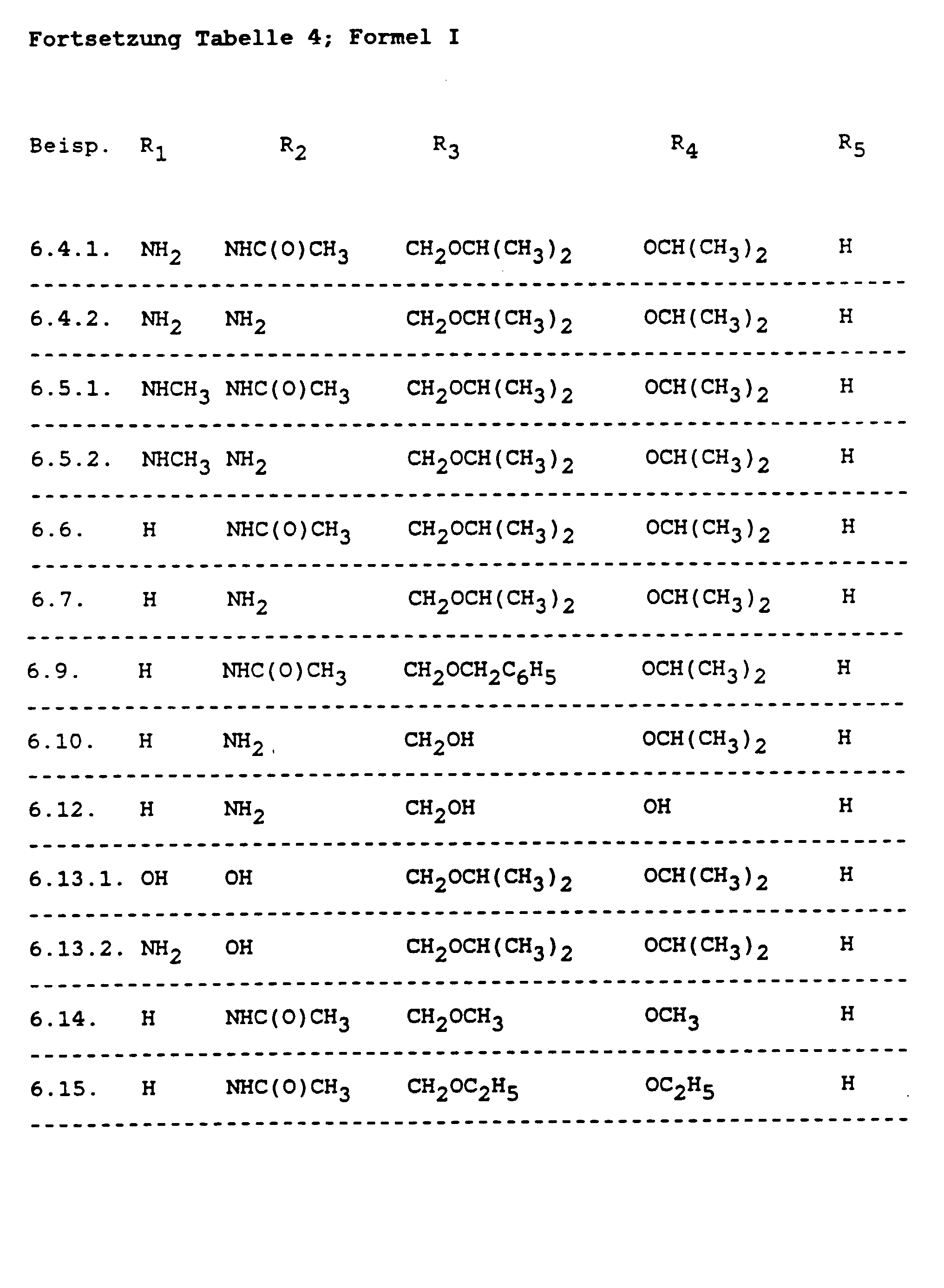

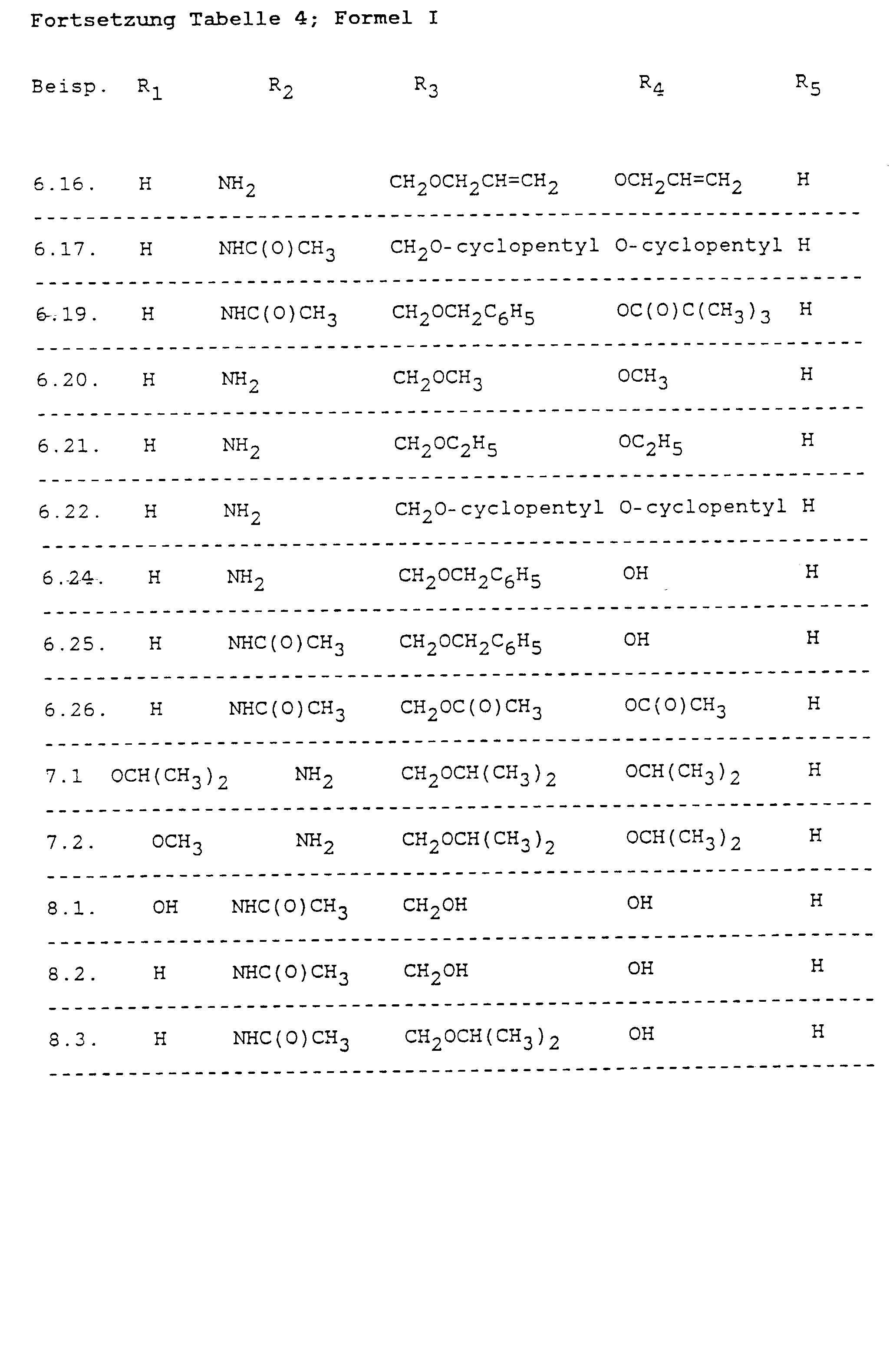

## Patentansprüche (Patentansprüche für folgende(n) Vertragsstaat(en): AT, BE, CH, DE, DK, FR, GB, IT, LI, LU, NL, SE)

1. Verbindungen der Formel I, 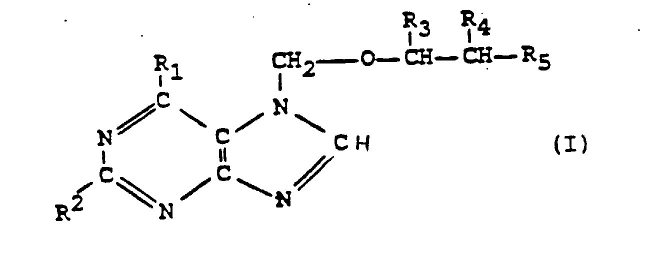 in der
R₁ Wasserstoff bedeutet,
R₂ Amino bedeutet,
R₃ C1-C3-Alkyl gegebenenfalls substituiert mit Hydroxy oder mit C1-C4-Acyloxy oder mit C1-C4-Alkoxy bedeutet,
R₄ Hydroxy oder C1-C4-Acyloxy oder C1-C4-Alkoxy bedeutet und
R₅ Wasserstoff bedeutet.

2. Verbindung der Formel 1 gemäß Anspruch 1, in der
R₁ Wasserstoff bedeutet,
R₂ Amino bedeutet,
R₃ Hydroxymethyl bedeutet,
R₄ Hydroxy bedeutet und
R₅ Wasserstoff bedeutet.

3. Verbindungen der Formel I, gemäß Anspruch 1 sowie deren physiologisch verträglichen Salze zur Prophylaxe oder Behandlung von Viruserkrankungen.

4. Verfahren zur Herstellung von Verbindungen der Formel I gemäß einem oder mehreren der Ansprüche 1 bis 3, dadurch gekennzeichnet, daß man
a) wenn in der Verbindung der Formel I R4 Hydroxy ist, eine Schutzgruppe A₁ in einer Verbindung der Formel II durch eine Hydroxygruppe ersetzt oder 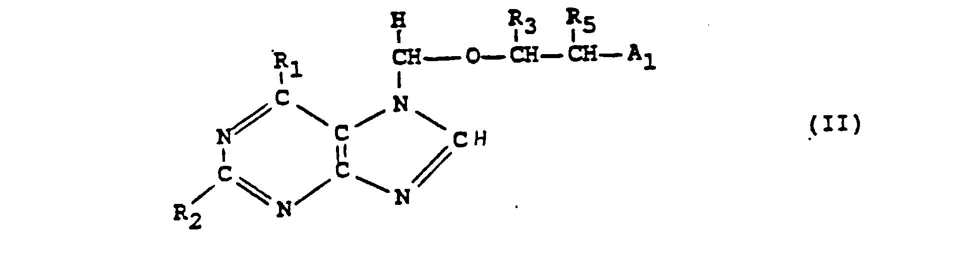
b) wenn in der Verbindung der Formel I R3 Hydroxyalkyl ist, eine Schutzgruppe A₂ in einer Verbindung der Formel III durch eine Hydroxygruppe ersetzt, 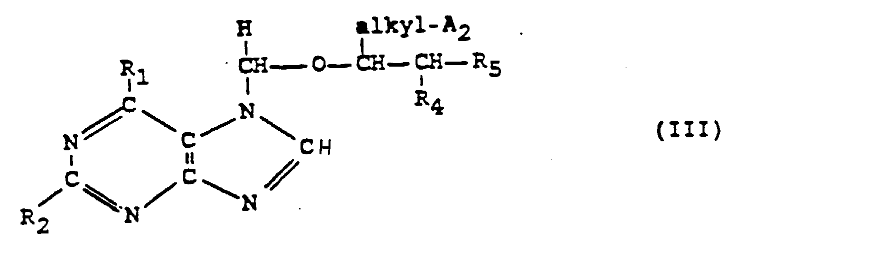
d) wenn in der Verbindung der Formel I R₃ Hydroxyalkyl ist und/oder R₄ Hydroxy ist, eine Schutzgruppe A₄ und/oder A₅ in einer Verbindung der Formel V durch eine Hydroxygruppe ersetzt oder 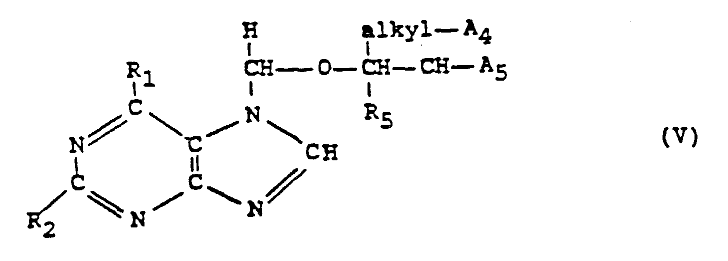
e) eine Verbindung der Formel VI, worin Y und Z Vorläufer der Gruppen R₁ bzw. R₂ sind, in eine Verbindung der Formel I umwandelt, worin R₁ und R₂ die genannten Bedeutungen haben oder 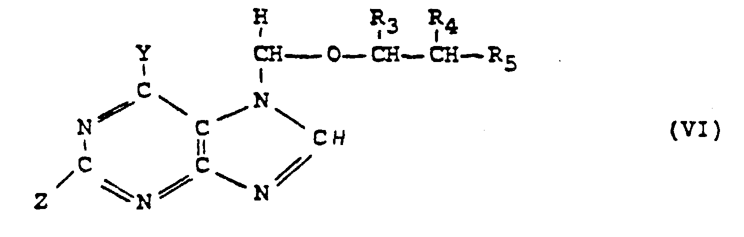
f) eine Verbindung der Formel VII 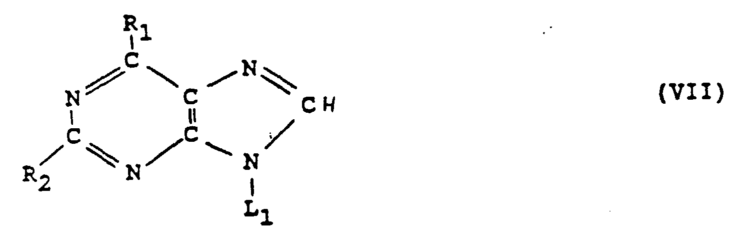 mit einer Verbindung der Formel VIII 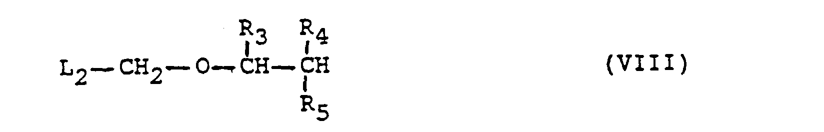 umsetzt, worin L₂ eine austretende Gruppe ist und L₁ Wasserstoff oder eine austretende Gruppe ist
g) eine Blockierungsgruppe von einer Verbindung der Formel I, worin einer oder beide Reste R₁ und R₂ blockiert sind, entfernt und insoweit das Produkt der Reaktion eine Base der Formel I ist, es gegebenenfalls in ein Säureadditionsprodukt dieser Base der Formel I umwandelt, oder insoweit das Produkt der Reaktion ein Salz einer Base der Formel I darstellt, es gegebenenfalls in seine Base oder in ein anderes Salz dieser Base umwandelt.

5. Arzneimittel mit einem Gehalt an mindestens einer Verbindung der Formel I gemäß einem oder mehreren der Ansprüche 1 bis 2.

6. Verwendung von Purinderivaten der Formel I gemäß einem oder mehreren der Ansprüche 1 bis 2 zur Herstellung von Arzneimitteln zur Behandlung von Viruserkrankungen.

7. Verfahren zur Herstellung von Arzneimitteln gemäß Anspruch 5, dadurch gekennzeichnet, daß man mindestens eine Verbindung der Formel I gemäß den Ansprüchen 1 bis 2 gegebenenfalls mit geeigneten Hilfs- und/oder Trägerstoffen in eine geeignete Darreichungsform bringt.

## Patentansprüche (Patentansprüche für folgende(n) Vertragsstaat(en): ES, GR)

1. Verfahren zur Herstellung von Verbindungen der Formel I, 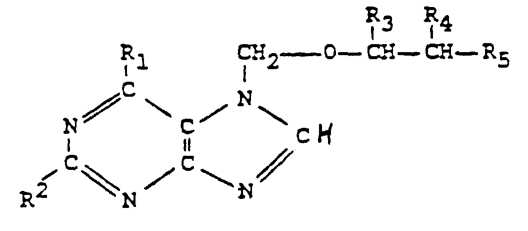 in der
R₁ Wasserstoff bedeutet,
R₂ Amino bedeutet,
R₃ C1-C3-Alkyl gegebenenfalls substituiert mit Hydroxy oder mit C1-C4-Acyloxy oder mit C1-C4-Alkoxy bedeutet,
R₄ Hydroxy oder C1-C4-Acyloxy oder C1-C4-Alkoxy bedeutet und
R₅ Wasserstoff bedeutet,
dadurch gekennzeichnet, daß man
a) wenn in der Verbindung der Formel I R4 Hydroxy ist, eine Schutzgruppe A₁ in einer Verbindung der Formel II durch eine Hydroxygruppe ersetzt oder 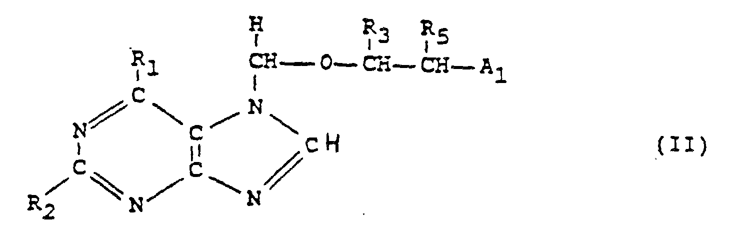
b) wenn in der Verbindung der Formel I R3 Hydroxyalkyl ist, eine Schutzgruppe A₂ in einer Verbindung der Formel III durch eine Hydroxygruppe ersetzt, 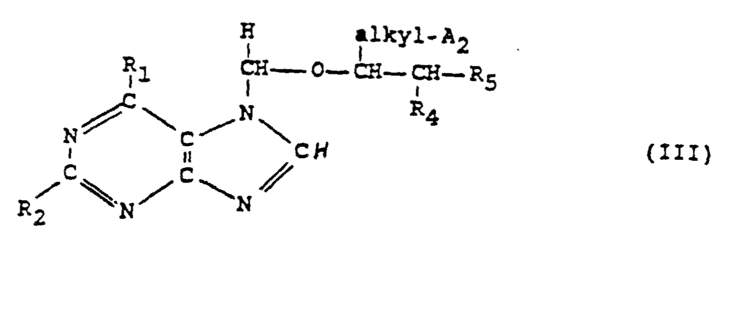
d) wenn in der Verbindung der Formel I R₃ Hydroxyalkyl ist und/oder R₄ Hydroxy ist, eine Schutzgruppe A₄ und/oder A₅ in einer Verbindung der Formel V durch eine Hydroxygruppe ersetzt oder 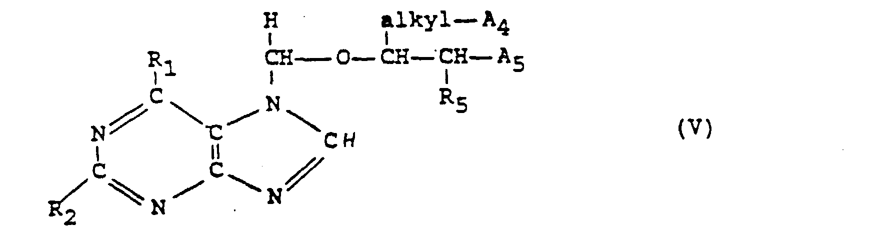
e) eine Verbindung der Formel VI, worin Y und Z Vorläufer der Gruppen R₁ bzw. R₂ sind, in eine Verbindung der Formel I umwandelt, worin R₁ und R₂ die genannten Bedeutungen haben oder 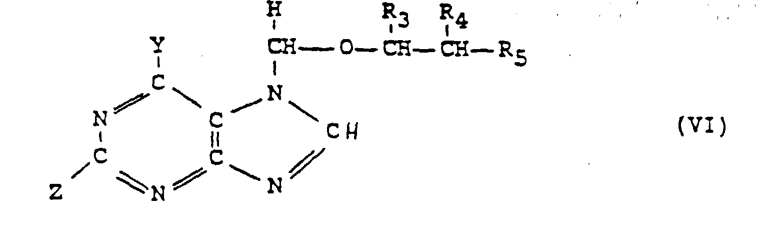
f) eine Verbindung der Formel VII 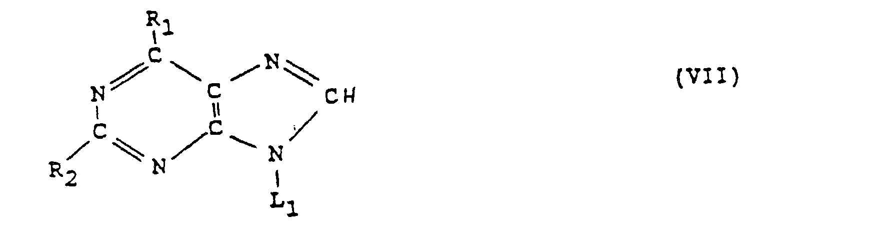 mit einer Verbindung der Formel VIII 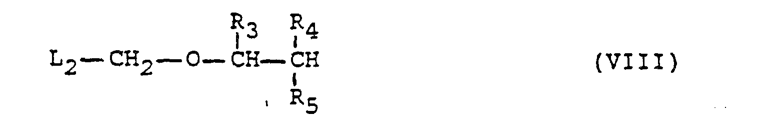 umsetzt, worin L₂ eine austretende Gruppe ist und L₁ Wasserstoff oder eine austretende Gruppe ist
g) eine Blockierungsgruppe von einer Verbindung der Formel I, worin einer oder beide Reste R₁ und R₂ blockiert sind, entfernt und insoweit das Produkt der Reaktion eine Base der Formel I ist, es gegebenenfalls in ein Säureadditionsprodukt dieser Base der Formel I umwandelt, oder insoweit das Produkt der Reaktion ein Salz einer Base der Formel I darstellt, es gegebenenfalls in seine Base oder in ein anderes Salz dieser Base umwandelt.

2. Verfahren zur Herstellung von Verbindungen der Formel I gemäß Anspruch 1, dadurch gekennzeichnet, daß
R₁ Wasserstoff bedeutet,
R₂ Amino bedeutet,
R₃ Hydroxymethyl bedeutet,
R₄ Hydroxy bedeutet und
R₅ Wasserstoff bedeutet.

3. Verfahren zur Herstellung von Verbindungen der Formel I, gemäß Anspruch 1 sowie deren physiologisch verträglichen Salze zur Prophylaxe oder Behandlung von Viruserkrankungen.

4. Arzneimittel mit einem Gehalt an mindestens einer Verbindung der Formel I gemäß einem oder mehreren der Ansprüche 1 bis 2.

5. Verwendung von Purinderivaten der Formel I gemäß einem oder mehreren der Ansprüche 1 bis 2 zur Herstellung von Arzneimitteln zur Behandlung von Viruserkrankungen.

6. Verfahren zur Herstellung von Arzneimitteln gemäß Anspruch 4, dadurch gekennzeichnet, daß man mindestens eine Verbindung der Formel I gemäß den Ansprüchen 1 bis 2 gegebenenfalls mit geeigneten Hilfs- und/oder Trägerstoffen in eine geeignete Darreichungsform bringt.

## Claims (Claims for the following Contracting State(s): AT, BE, CH, DE, DK, FR, GB, IT, LI, LU, NL, SE)

1. A compound of the formula I 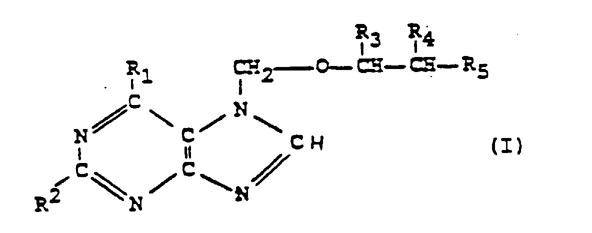 in which
R₁ is hydrogen,
R₂ is amino,
R₃ is C₁-C₃-alkyl, optionally substituted by hydroxyl or by C₁-C₄-acyloxy or by C₁-C₄-alkoxy,
R₄ is hydroxyl or C₁-C₄-acyloxy or C₁-C₄-alkoxy and
R₅ is hydrogen.

2. A compound of the formula I as claimed in claim 1, in which
R₁ is hydrogen,
R₂ is amino,
R₃ is hydroxymethyl,
R₄ is hydroxyl and
R₅ is hydrogen.

3. A compound of the formula I as claimed in claim 1 or its physiologically tolerable salts for the prophylaxis or treatment of viral diseases.

4. A process for the preparation of a compound of the formula I as claimed in one or more of claims 1 to 3, which comprises
a) if in the compound of the formula I R₄ is hydroxyl, replacing a protecting group A₁ in a compound of the formula II 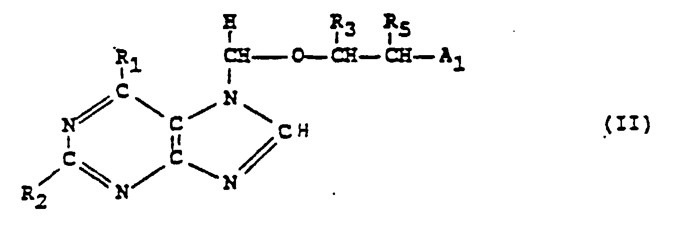 by a hydroxyl group, or
b) if in the compound of the formula I R₃ is hydroxyalkyl, replacing a protecting group A₂ in a compound of the formula III 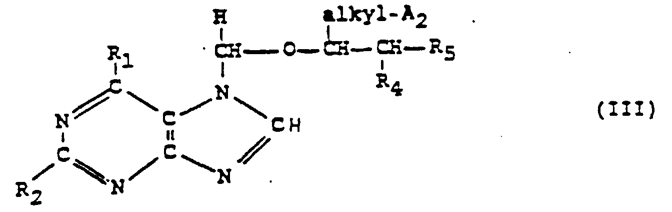
c) if in the compound of the formula I R₃ is hydroxyalkyl and/or R₄ is hydroxyl replacing a protecting group A₄ and/or A₅ in a compound of the formula V 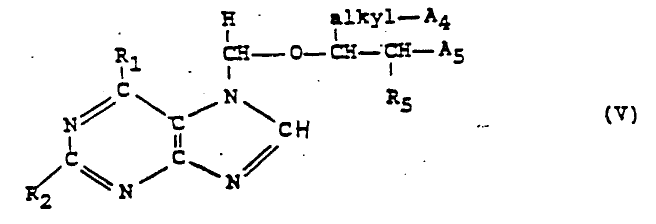 by a hydroxyl group, or
d) converting a compound of the formula VI 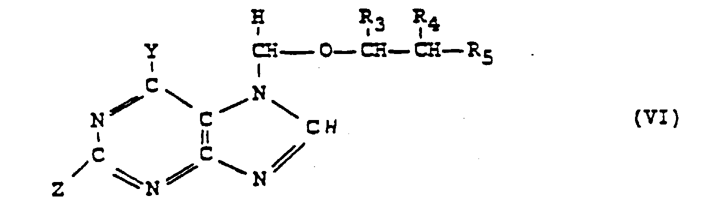 in which Y and Z are precursors of the groups R₁ and R₂ into a compound of the formula I in which R₁ and R₂ have the meanings mentioned, or
e) reacting a compound of the formula VII 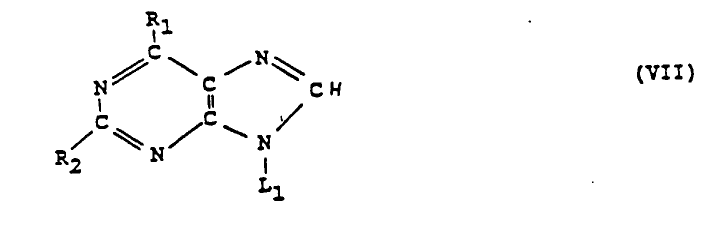 with a compound of the formula VIII 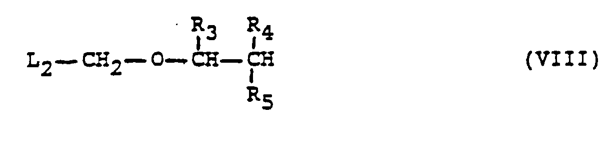 in which L₂ is a leaving group and L₁ is hydrogen or a leaving group,
f) removing a blocking group from a compound of the formula I in which one or both radicals R₁ and R₂ are blocked, and if the product of the reaction is a base of the formula I, optionally converting it into an acid addition product of this base of the formula I, or if the product of the reaction is a salt of a base of the formula I, optionally converting it into its base or into another salt of this base.

5. A pharmaceutical containing at least one compound of the formula I as claimed in one or more of claims 1 to 2.

6. The use of purine derivatives of the formula I as claimed in one or more of claims 1 to 2 for the production of pharmaceuticals for the treatment of viral diseases.

7. A process for the production of pharmaceuticals as claimed in claim 5, which comprises bringing at least one compound of the formula I as claimed in claims 1 to 2 into a suitable administration form, if appropriate with suitable auxiliaries and/or excipients.

## Claims (Claims for the following Contracting State(s): ES, GR)

1. A process for the preparation of a compound of the formula I 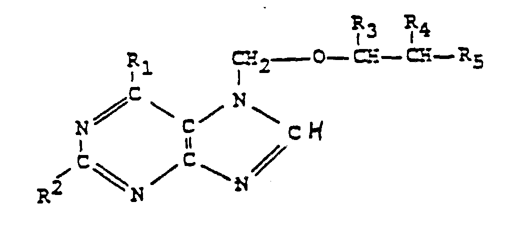 in which
R₁ is hydrogen,
R₂ is amino,
R₃ is C₁-C₃-alkyl, optionally substituted by hydroxyl or by C₁-C₄-acyloxy or by C₁-C₄-alkoxy,
R₄ is hydroxyl or C₁-C₄-acyloxy or C₁-C₄-alkoxy and
R₅ is hydrogen,
which comprises
a) if in the compound of the formula I R₄ is hydroxyl, replacing a protecting group A₁ in a compound of the formula II 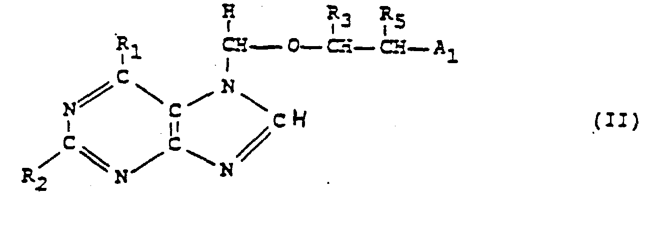 by a hydroxyl group, or
b) if in the compound of the formula I R₃ is hydroxyalkyl, replacing a protecting group A₂ in a compound of the formula III 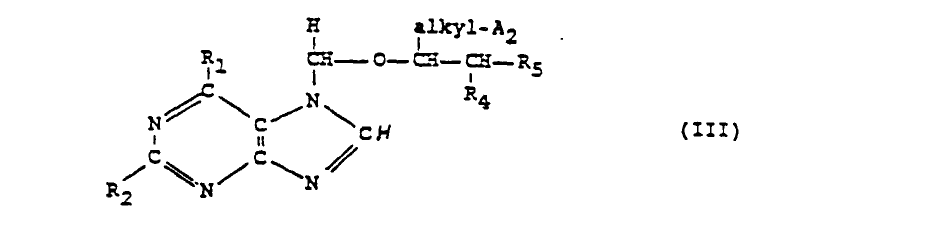 by a hydroxyl group, or
c) if in the compound of the formula I R₃ is hydroxyalkyl and/or R₄ is hydroxyl, replacing a protecting group A₄ and/or A₅ in a compound of the formula V 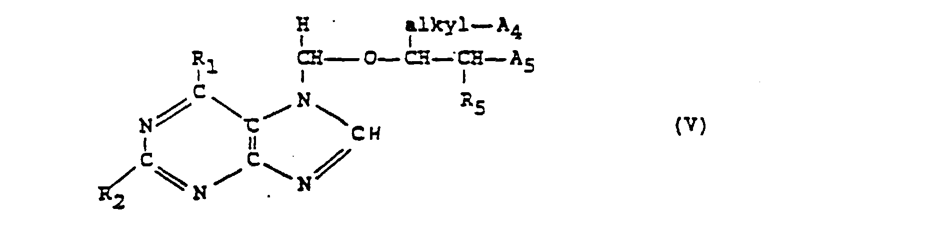 by a hydroxyl group, or
d) converting a compound of the formula VI 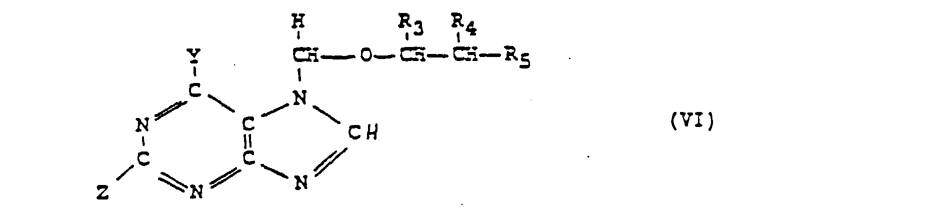 in which Y and Z are precursors of the groups R₁ and R₂ into a compound of the formula I in which R₁ and R₂ have the meanings mentioned, or
e) reacting a compound of the formula VII 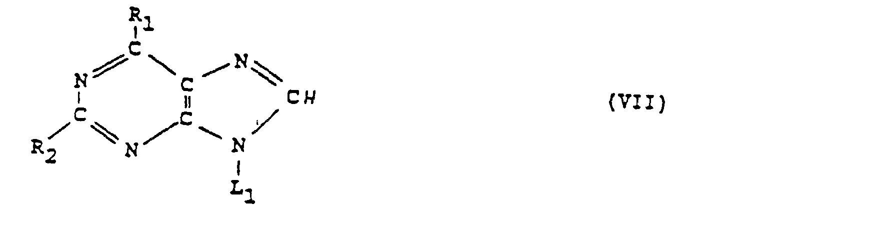 with a compound of the formula VIII 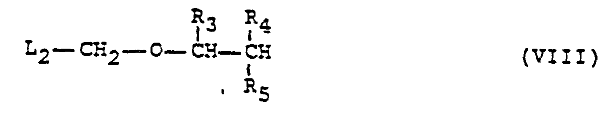 in which L₂ is a leaving group and L₁ is hydrogen or a leaving group,
f) removing a blocking group from a compound of the formula I in which one or both radicals R₁ and R₂ are blocked, and if the product of the reaction is a base of the formula I, optionally converting it into an acid addition product of this base of the formula I, or if the product of the reaction is a salt of a base of the formula I, optionally converting it into its base or into another salt of this base.

2. The process for the preparation of a compound of the formula I as claimed in claim 1, wherein
R₁ is hydrogen,
R₂ is amino,
R₃ is hydroxymethyl,
R₄ is hydroxyl and
R₅ is hydrogen.

3. The process for the preparation of a compound of the formula I as claimed in claim 1 or its physiologically tolerable salts for the prophylaxis or treatment of viral diseases.

4. A pharmaceutical containing at least one compound of the formula I as claimed in one or more of claims 1 to 2.

5. The use of purine derivatives of the formula I as set forth in one or more of claims 1 to 2 for the production of pharmaceuticals for the treatment of viral diseases.

6. A process for the production of pharmaceuticals as claimed in claim 4, which comprises bringing at least one compound of the formula I as claimed in claims 1 to 2 into a suitable administration form, if appropriate with suitable auxiliaries and/or excipients.

## Revendications (Revendications pour l'(les) Etat(s) contractant(s) suivant(s): AT, BE, CH, DE, DK, FR, GB, IT, LI, LU, NL, SE)

1. Composés de Formule I, 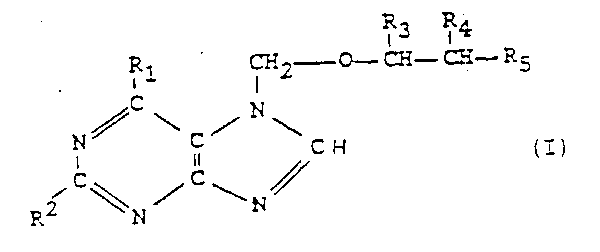 dans laquelle
R₁ représente un atome d'hydrogène,
R₂ représente un groupe amino,
R₃ représente un groupe alkyle en C₁-C₃ éventuellement substitué par un groupe hydroxy ou acyloxy en C₁-C₄ ou alcoxy en C₁-C₄,
R₄ représente un groupe hydroxy ou acyloxy en C₁-C₄ ou alcoxy en C₁-C₄ et
R₅ représente un atome d'hydrogène.

2. Composés de Formule selon la revendication 1, dans laquelle
R₁ représente un atome d'hydrogène,
R₂ représente un groupe amino,
R₃ représente un groupe hydroxyméthyle,
R₄ représente un groupe hydroxy,
R₅ représente un atome d'hydrogène.

3. Composés de Formule I selon la revendication 1, ainsi que leurs sels physiologiquement acceptables pour la prophylaxie ou le traitement de maladies virales.

4. Procédé de fabrication de composés de Formule I selon une ou plusieurs des revendications 1 à 3, caractérisé en ce que,
a) lorsque dans le composé de Formule I, R₄ est un groupe hydroxy, l'on remplace un groupe protecteur A₁ dans un composé de Formule II par un groupe hydroxy, ou 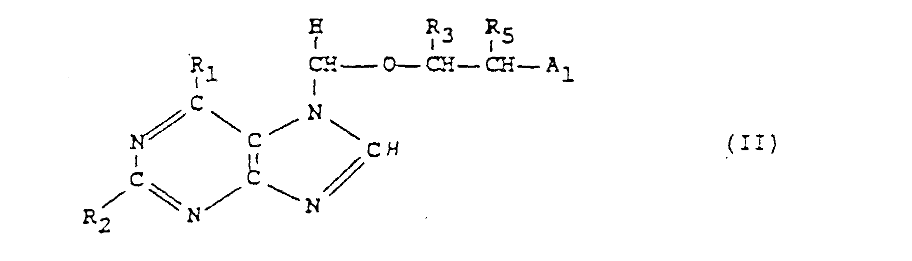
b) lorsque dans le composé de Formule I, R₃ est un groupe hydroxyalkyle, l'on remplace un groupe protecteur A₂ dans un composé de Formule III par un groupe hydroxy, ou 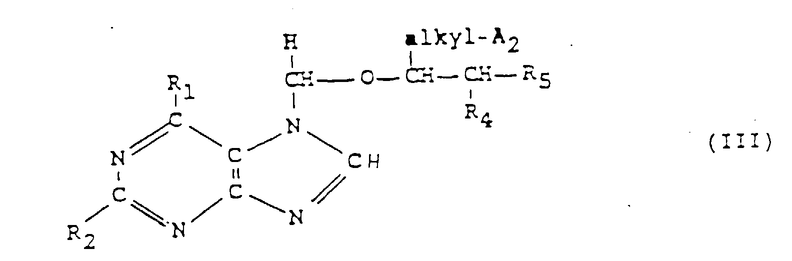
d) lorsque dans le composé de Formule I, R₃ est un groupe hydroxyalkyle et/ou R₄ est un groupe hydroxy, l'on remplace un groupe protecteur A₄ et/ou A₅ dans un composé de Formule V par un groupe hydroxy, ou 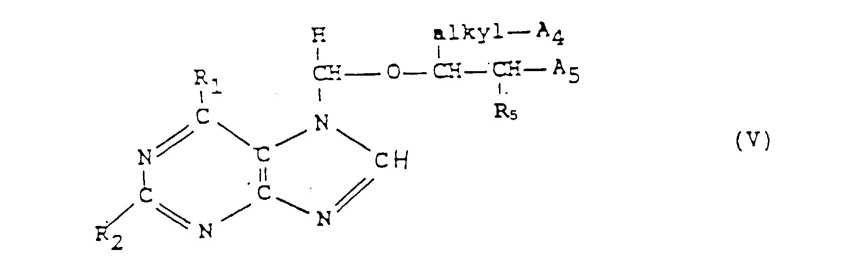
e) l'on transforme un composé de Formule VI dans laquelle Y et Z sont des précurseurs des groupes R₁ et R₂, en un composé de Formule I, dans laquelle R₁ et R₂ ont les significations mentionnées ci-dessus, ou 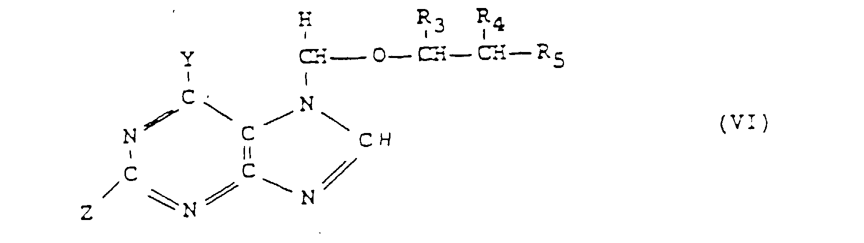
f) l'on fait réagir un composé de Formule VII 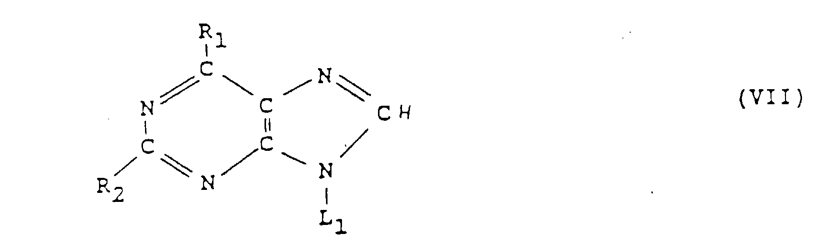 avec un composé de Formule VIII 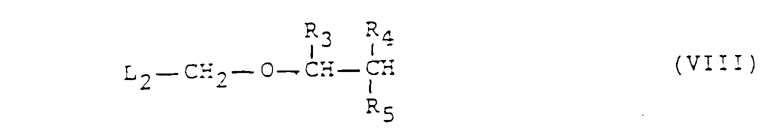 dans laquelle L₂ est un groupe partant et L₁ est un atome d'hydrogène ou un groupe partant
g) l'on élimine un groupe de blocage d'un composé de Formule I, dans laquelle l'un ou les deux résidus R₁ et R₂ sont bloqués, et dans la mesure où le produit de la réaction est une base de Formule I, on le transforme éventuellement en un produit d'addition de cette base de Formule I avec un acide, ou dans la mesure où le produit de la réaction est un sel d'une base de Formule I, on le transforme éventuellement en sa base ou en un autre sel de cette base.

5. Médicament contenant au moins un composé de Formule I selon une ou plusieurs des revendications 1 à 2.

6. Utilisation de dérivés de purines de Formule I selon une ou plusieurs des revendications 1 à 2 pour produire des médicaments pour le traitement de maladies virales.

7. Procédé de fabrication de médicaments selon la revendication 5, caractérisé en ce que l'on met au moins un composé de Formule I selon les revendications 1 à 2 sous une forme de présentation appropriée, avec éventuellement des adjuvants et/ou des véhicules.

## Revendications (Revendications pour l'(les) Etat(s) contractant(s) suivant(s): ES, GR)

1. Procédé de fabrication de composés de Formule I 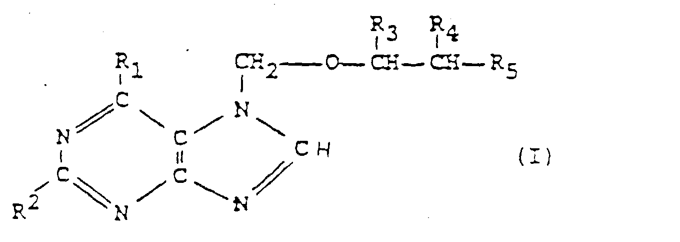 dans laquelle
R₁ représente un atome d'hydrogène,
R2 représente un groupe amino,
R₃ représente un groupe alkyle en C₁-C₃ éventuellement substitué par un groupe hydroxy ou acyloxy en C₁-C₄ ou alcoxy en C₁-C₄,
R₄ représente un groupe hydroxy ou acyloxy en C₁-C₄ ou alcoxy en C₁-C₄ et
R₅ représente un atome d'hydrogène, caractérisé en ce que,
a) lorsque dans le composé de Formule I, R₄ est un groupe hydroxy, l'on remplace un groupe protecteur A₁ dans un composé de Formule II par un groupe hydroxy, ou 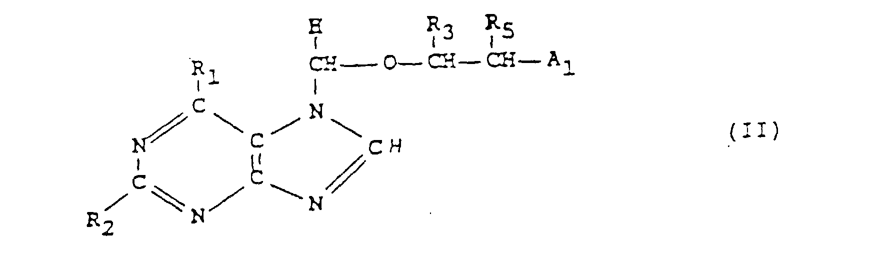
b) lorsque dans le composé de Formule I, R₃ est un groupe hydroxyalkyle, l'on remplace un groupe protecteur A₂ dans un composé de Formule III par un groupe hydroxy, ou 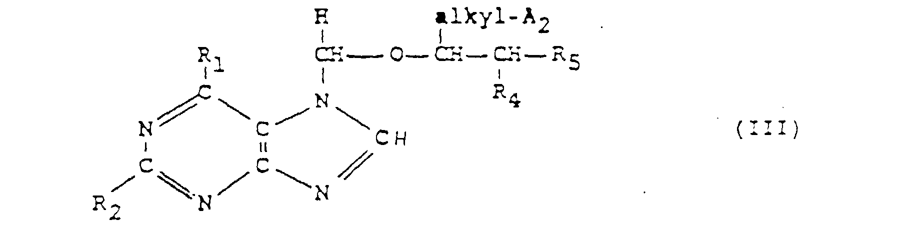
d) lorsque dans le composé de Formule I, R₃ est un groupe hydroxyalkyle et/ou R₄ est un groupe hydroxy, l'on remplace un groupe protecteur A₄ et/ou A₅ dans un composé de Formule V par un groupe hydroxy ou 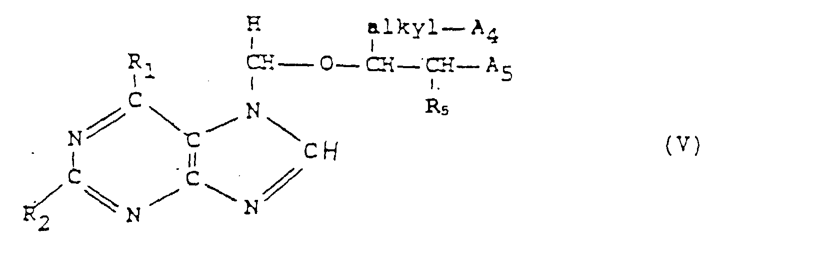
e) l'on transforme un composé de Formule VI, dans laquelle Y et Z sont des précurseurs des groupes R₁ en R₂, en un composé de Formule I, dans laquelle R₁ et R₂ ont les significations mentionnées ci-dessus, ou 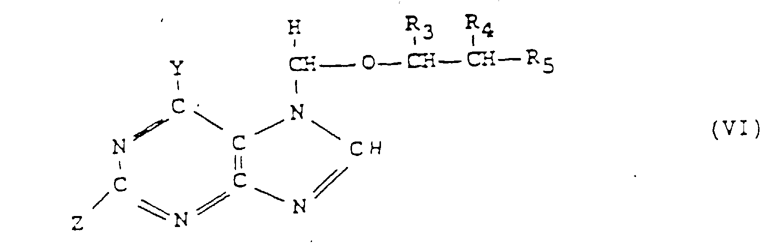
f) l'on fait réagir un composé de Formule VII 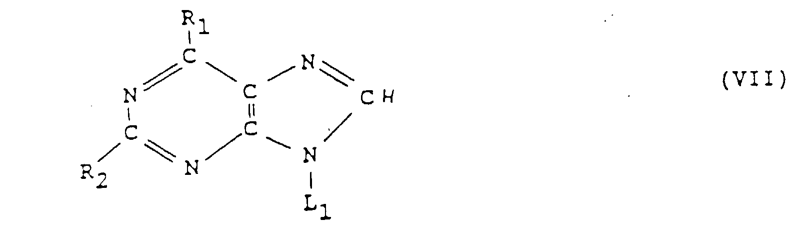 avec un composé de Formule VIII 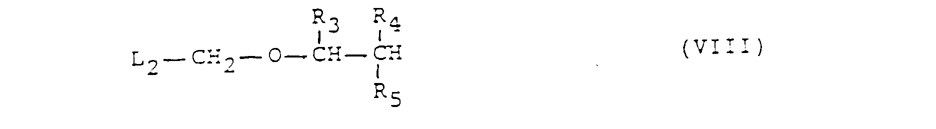 dans laquelle L₂ est un groupe partant et L₁ est un atome d'hydrogène ou un groupe partant
g) l'on élimine un groupe de blocage d'un composé de Formule I, dans laquelle l'un ou les deux résidus R₁ et R₂ sont bloqués, et dans la mesure où le produit de la réaction est une base de Formule I, on le transforme éventuellement en un produit d'addition de cette base de Formule I avec un acide, ou dans la mesure où le produit de la réaction est un sel d'une base de Formule I, on le transforme éventuellement en sa base ou en un autre sel de cette base.

2. Procédé de fabrication de composés de Formule I selon la revendication 1, caractérisé en ce que,
R₁ représente un atome d'hydrogène,
R₂ représente un groupe amino,
R₃ représente un groupe hydroxyméthyle,
R₄ représente un groupe hydroxy,
R₅ représente un atome d'hydrogène.

3. Procédé de fabrication de composés de Formule I selon la revendication 1, ainsi que de leurs sels physiologiquement acceptables pour la prophylaxie ou le traitement de maladies virales.

4. Médicament contenant au moins un composé de Formule I selon une ou plusieurs des revendications 1 à 2.

5. Utilisation de dérivés de purines de Formule I selon une ou plusieurs des revendications 1 à 2 pour produire des médicaments pour le traitement des maladies virales.

6. Procédé de fabrication de médicaments selon la revendication 4, caractérisé en ce que, l'on met au moins un composé de Formule I selon les revendications 1 à 2 sous une forme de présentation appropriée, avec éventuellement des adjuvants et/ou des véhicules.
